# EUROPEAN PATENT SPECIFICATION

(11) **EP 3 061 470 B1**
(45) Date of publication and mention of the grant of the patent: **16.12.2020**
(21) Application number: 14855362.1
(22) Date of filing: 08.10.2014
(51) Int. Cl.: A61L 31/00, A61L 29/00, A61M 25/09

(54) **METAL MEDICAL DEVICE HAVING LUBRICITY AND LOW PROTEIN ADSORPTION PROPERTIES AND/OR LOW CELL ADSORPTION PROPERTIES, AND METHOD FOR PRODUCING SAME**
MEDIZINISCHE METALLVORRICHTUNG MIT SCHMIERFÄHIGKEIT UND NIEDRIGER EIWEISSADSORPTION UND/ODER NIEDRIGER ZELLADSORPTION SOWIE VERFAHREN ZUR HERSTELLUNG DAVON
DISPOSITIF MÉDICAL MÉTALLIQUE AYANT UNE LUBRICITÉ ET DES CARACTÉRISTIQUES DE FAIBLE ADSORPTION DE PROTÉINES ET/OU DES CARACTÉRISTIQUES DE FAIBLE ADSORPTION DE CELLULES, ET SON PROCÉDÉ DE PRODUCTION

(30) Priority: 21.10.2013 JP 2013218590; 29.08.2014 JP 2014176046
(43) Date of publication of application: 31.08.2016
(73) Proprietor: Sumitomo Rubber Industries, Ltd., Kobe-shi, Hyogo 651-0072 (JP)
(72) Inventor: MINAGAWA Yasuhisa, Kobe-shi Hyogo 651-0072 (JP); NAKASHITA Takefumi, Kobe-shi Hyogo 651-0072 (JP)
(74) Representative: Manitz Finsterwald Patent- und Rechtsanwaltspartnerschaft mbB
(86) International application number: PCT/JP2014/076887
(87) International publication number: WO 2015/060129

(56) References cited:
- WO-A1-01/60923
- WO-A1-2008/023604
- JP-A- H10 231
- JP-A- H0 747 120
- JP-A- H04 250 158
- JP-A- H05 115 541
- JP-A- H10 330 383
- JP-A- 2001 029 452
- JP-A- 2002 502 286
- JP-A- 2006 061 273
- US-A1- 2003 215 649
- US-A1- 2011 086 234
- US-A1- 2011 086 234
- US-A1- 2011 159 101
- Gelest Inc.: "Hydrophobicity, Hydrophilicity and Silane Surface Modification", , 24 May 2011 (2011-05-24), pages 1-82, XP055098863, Retrieved from the Internet: URL:https://acs.expoplanner.com/files/acsf all13/ExhibFiles/1047_1100_HydrophobicityC atalogUS_5-24-11.pdf [retrieved on 2014-01-28]

## Description

### TECHNICAL FIELD

The present invention relates to a metal medical device having lubricity and low protein adsorption properties and/or low cell adsorption properties, and a method for preparing the same.

### BACKGROUND ART

Guide wires, stents, and the like are inserted into and optionally placed in blood vessels, respiratory tracts, urethra, or other body cavities or tissues. When a medical device such as a guide wire or stent is inserted into the body in such a case, the medical device may damage the tissue or the like in the body and produce inflammation or cause pain to the patient. To ameliorate these problems, it has been desired to improve the sliding properties of medical devices intended to be inserted into the body.

Moreover, stents and the like which are placed in the body for a long period of time are required to be prevented, as far as possible, from adsorbing proteins and cells on their surface because the adsorption of proteins or cells on the surface can lead to problems such as the formation of a blood clot clogging a blood vessel.

To ameliorate the above problems, a method has been proposed in which the surface of a medical device such as guide wire or stent is coated with a hydrophilic resin, a fluororesin or the like.
US 2011/0086234 A1 discloses a NO-releasing sol.gel coating formed from a sol precursor solution comprising a backbone alkoxysilane and a diazeniumdiolate-modified alkoxysilane. US 2011/159101 A1 discloses hydrophilic polymers having pendent silyl ether-containing groups such as poly(vinylpyrrolidone)-silane and polymeric matrices formed from these polymers, which can be used in association with an implantable or insertable medical device.

### SUMMARY OF INVENTION

### TECHNICAL PROBLEM

As described above, various methods have been tried to impart lubricity to the surface of medical devices to improve the sliding properties thereof. However, all the methods only allow the surface of medical devices to be coated with a resin or to be cured after the coating. Especially in the case where the surface of the medical device is made of a metal, since the coating resin is not firmly bonded to the surface of the medical device, it can be easily peeled or removed from the surface of the medical device, with the result that unfortunately the sliding properties of the medical device are deteriorated. Another problem is that proteins or cells gradually adhere to the surface of the indwelling medical device. Accordingly, it has been desired to develop metal medical devices in which deterioration of sliding properties and deterioration of low protein or cell adsorption/adhesion properties are suppressed.

The present invention aims to solve the above problems and provide a metal medical device such as a guide wire or stent in which a lubricant layer is firmly bonded to the surface of the metal medical device to impart lubricity and low protein adsorption properties and/or low cell adsorption properties to the surface and, further, to improve the durability of the lubricant layer on the surface of the metal medical device, thereby suppressing deterioration of sliding properties and deterioration of low protein adsorption properties and/or low cell adsorption properties, as well as a method for preparing such a metal medical device.

### SOLUTION TO PROBLEM

The present invention relates to a metal medical device, having lubricity and at least one of low protein adsorption properties or low cell adsorption properties and having a surface at least partially treated with at least one silane compound compound selected from the group consisting of a silane compound represented by the following formula (1), a silane compound represented by the following formula (2), a silane compound represented by the following formula (8), a silane compound represented by the following formula (9), a silane compound represented by the following formula (16), a silane compound containing a linking unit A represented by the formula (17) and a linking unit B represented by the formula (18),
a silane compound represented by the following formula (23), and
a silane compound represented by the following formula (24). 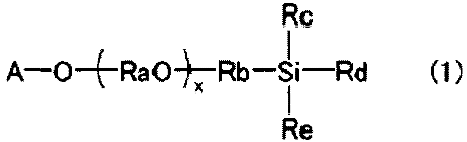 wherein A represents a hydrogen atom, a linear or branched C1-C3 alkyl group, or a linear or branched C1-C3 acyl group; Ra represents a linear or branched C1-C3 alkylene group; Rb represents a linear or branched C1-C5 alkylene group; Rc, Rd, and Re are the same as or different from one another, and each represent a halogen atom, a hydroxy group, a linear or branched C1-C3 alkyl group, or a linear or branched C1-C3 alkoxy group, provided that at least one of Rc, Rd, and Re is a halogen atom, a hydroxy group, or a linear or branched C1-C3 alkoxy group; and x represents the number of oxyalkylene repeating units each represented by RaO and is an integer of 2 to 500, 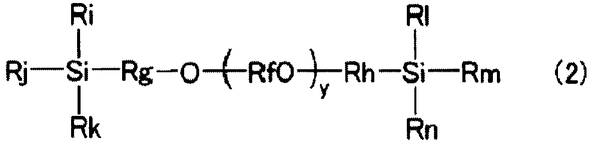 wherein Rf represents a linear or branched C1-C3 alkylene group; Rg and Rh are the same as or different from one another, and each represent a linear or branched C1-C5 alkylene group; Ri, Rj, Rk, Rl, Rm, and Rn are the same as or different from one another, and each represent a halogen atom, a hydroxy group, a linear or branched C1-C3 alkyl group, or a linear or branched C1-C3 alkoxy group, provided that at least one of Ri, Rj, Rk, Rl, Rm, and Rn is a halogen atom, a hydroxy group, or a linear or branched C1-C3 alkoxy group; and y represents the number of oxyalkylene repeating units each represented by RfO and is an integer of 2 to 500, 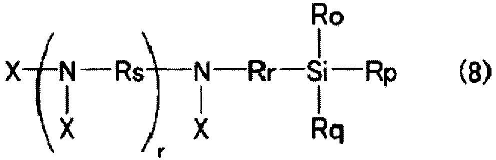 wherein Ro, Rp, and Rq are the same as or different from one another, and each represent a halogen atom, a hydroxy group, a linear or branched C1-C3 alkyl group, or a linear or branched C1-C3 alkoxy group, provided that at least one of Ro, Rp, and Rq is a halogen atom, a hydroxy group, or a linear or branched C1-C3 alkoxy group; Rr and Rs are the same as or different from one another, and each represent a linear or branched C1-C7 alkylene group; each X is the same or different and represents a metal salt-containing hydrophilic group; and r represents 0 or 1, 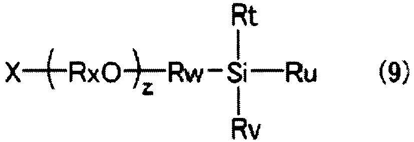 wherein Rt, Ru, and Rv are the same as or different from one another, and each represent a halogen atom, a hydroxy group, a linear or branched C1-C3 alkyl group, or a linear or branched C1-C3 alkoxy group, provided that at least one of Rt, Ru, and Rv is a halogen atom, a hydroxy group, or a linear or branched C1-C3 alkoxy group; Rw represents a linear or branched C1-C5 alkylene group; Rx represents a linear or branched C1-C3 alkylene group; each X is the same or different and represents a metal salt-containing hydrophilic group; and z represents the number of oxyalkylene repeating units each represented by RxO and is an integer of 2 to 500, 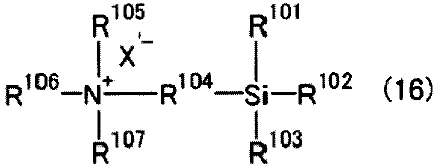 wherein R¹⁰¹, R¹⁰², and R¹⁰³ are the same as or different from one another, and each represent a halogen atom, a hydroxy group, a linear or branched C1-C3 alkyl group, or a linear or branched C1-C3 alkoxy group, provided that at least one of R¹⁰¹, R¹⁰², and R¹⁰³ is a halogen atom, a hydroxy group, or a linear or branched C1-C3 alkoxy group; R¹⁰⁴ represents a linear or branched C1-C7 alkylene group; R¹⁰⁵, R¹⁰⁶, and R¹⁰⁷ are the same as or different from one another, and each represent a hydrogen atom or a linear or branched C1-C5 alkyl group optionally containing an ether linkage; and X' represents a halogen atom, 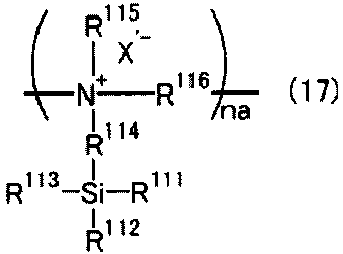 wherein R¹¹¹, R¹¹², and R¹¹³ are the same as or different from one another, and each represent a halogen atom, a hydroxy group, a linear or branched C1-C3 alkyl group, or a linear or branched C1-C3 alkoxy group, provided that at least one of R¹¹¹, R¹¹², and R¹¹³ is a halogen atom, a hydroxy group, or a linear or branched C1-C3 alkoxy group; R¹¹⁴ represents a linear or branched C1-C7 alkylene group; R¹¹⁵ represents a hydrogen atom or a linear or branched C1-C5 alkyl group optionally containing an ether linkage; R¹¹⁶ represents a linear or branched C1-C5 alkylene group; X' represents a halogen atom; and na represents an integer of 1 or more, 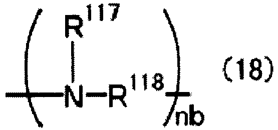 wherein R¹¹⁷ represents a hydrogen atom or a linear or branched C1-C5 alkyl group optionally containing an ether linkage; R¹¹⁸ represents a linear or branched C1-C5 alkylene group; and nb represents an integer of 1 or more, 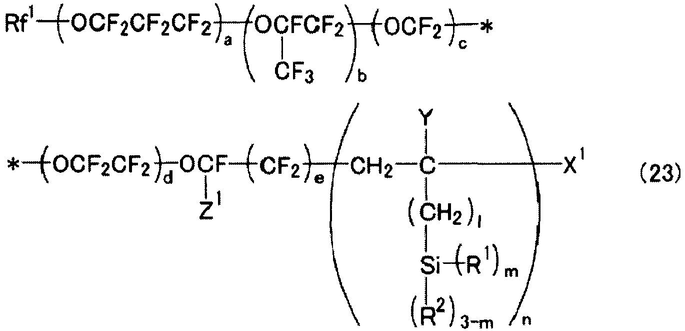 wherein Rf¹ represents a perfluoroalkyl group; Z¹ represents fluorine or a trifluoromethyl group; a, b, c, d, and e are the same as or different from one another, and each represent an integer of 0 or 1 or more, provided that a + b + c + d + e is 1 or more and the order of the repeating units parenthesized by subscripts a, b, c, d, and e occurring in the above formula is not limited to that shown; Y represents hydrogen or a C1-C4 alkyl group; X¹ represents hydrogen, bromine, or iodine; R¹ represents a hydroxy group or a hydrolyzable substituent; R² represents hydrogen or a monovalent hydrocarbon group; 1 represents 0, 1, or 2; m represents 1, 2, or 3; and n represents an integer of 1 or more. Please note that the two ends marked by * are directly bonded to each other, 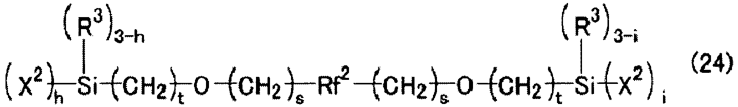 wherein Rf² represents a divalent group that contains a unit represented by -(CₖF₂ₖ)O- where k is an integer of 1 to 6, and has a non-branched linear perfluoropolyalkylene ether structure; each R³ is the same or different and represents a monovalent C1-C8 hydrocarbon group; each X² is the same or different and represents a hydrolyzable group or a halogen atom; each s is the same or different and represents an integer of 0 to 2; each t is the same or different and represents an integer of 1 to 5; and h and i are the same as or different from one another, and each represent 2 or 3.

The metal salt-containing hydrophilic group is preferably an alkali metal salt-containing hydrophilic group or an alkaline earth metal salt-containing hydrophilic group.

The halogen salt-containing hydrophilic group is preferably a chlorine salt-containing hydrophilic group.

The fluorine-containing hydrophobic group is preferably a perfluorooxyalkylene group.

The present invention also relates to a method for preparing a metal medical device having lubricity and at least one of low protein adsorption properties or low cell adsorption properties, the method including treating a surface of a metal medical device with a silane compound containing a functional group that is a polyoxyalkylene group, a metal salt-containing hydrophilic group, a halogen salt-containing hydrophilic group, or a fluorine-containing hydrophobic group, further comprising holding a surface-treated metal medical device obtained through the treatment step at a humidity of 50% or higher for 20 to 60 hours.

The treatment step is preferably carried out at a pH of 5 or lower.

### ADVANTAGEOUS EFFECTS OF INVENTION

According to the present invention, since the surface of a metal medical device is treated with a silane compound according to claim 1, the silane compound is chemically bonded to the hydroxy groups on the surface of the metal medical device, which makes it possible to impart lubricity and low protein adsorption properties and/or low cell adsorption properties to the surface of the metal medical device and, further, to improve the durability of the lubricant layer on the surface, thereby suppressing deterioration of the sliding properties of the metal medical device even when stress such as rubbing by a hand is applied. Furthermore, the lubricant layer can be prevented from being washed away and removed during the long indwelling time, and thus deterioration of low protein adsorption properties and/or low cell adsorption properties can be suppressed.

### DESCRIPTION OF EMBODIMENTS

The metal medical device having lubricity and low protein adsorption properties and/or low cell adsorption properties of the present invention has a surface at least partially treated with a silane compound according to claim 1.

Lubricant layers formed on the surface of metal medical devices by conventional surface treatment or coating methods are not chemically bonded to the surface and can be easily peeled or removed by application of stress such as rubbing by a hand. Thus, they are disadvantageous in terms of durability and maintaining sliding properties. Another problem is that these lubricant layers will be washed away and removed during the long indwelling time, so that low protein adsorption properties and/or low cell adsorption properties can be deteriorated. In contrast, in the metal medical device having lubricity and low protein adsorption properties and/or low cell adsorption properties of the present invention, since it is treated with the functional group-containing silane compound, the silane compound and the hydroxy groups on the surface of the metal medical device are chemically bonded to each other by a dehydration or deoxygenation condensation reaction therebetween. Thus, the lubricant layer on the surface of the metal medical device can be prevented from being peeled or removed due to stress such as rubbing, and thus deterioration of the sliding properties of the metal medical device can be prevented. Furthermore, the lubricant layer can be prevented from being washed away and removed during the long indwelling time, and thus deterioration of low protein adsorption properties and/or low cell adsorption properties can also be suppressed.

Moreover, in the case of a silane compound according to claim 1, such a functional group has high affinity particularly with water and thus can hold a large number of water molecules therearound, thereby causing fluid lubrication. Therefore, treatment with such a functional group-containing silane compound allows the metal medical device to have remarkably improved sliding properties. Furthermore, the lubricant layer can be prevented from being washed away and removed during the long indwelling time, and thus deterioration of low protein adsorption properties and/or low cell adsorption properties can also be suppressed.

On the other hand, in the case of a silane compound containing a fluorine-containing hydrophobic group according to claim 1, which can remarkably reduce the surface tension, treatment with such a silane compound containing a fluorine-containing hydrophobic group greatly reduces aggregation of molecules between the surface of the metal medical device and an object in contact with the surface (e.g., in the case where the metal medical device is a guide wire, the inner surface of a catheter, somatic cells or the like), thereby allowing the metal medical device to have greatly improved sliding properties. Furthermore, the lubricant layer can be prevented from being washed away and removed during the long indwelling time, and thus deterioration of low protein adsorption properties and/or low cell adsorption properties can also be suppressed.

The metal medical device having lubricity and low protein adsorption properties and/or low cell adsorption properties of the present invention has a surface treated with the silane compound at least at a portion where lubricity and low protein adsorption properties and/or low cell adsorption properties are required. The entire surface of the metal medical device may be treated with the silane compound.

The silane compound is a polyoxyalkylene group, a metal salt-containing hydrophilic group, a halogen salt-containing hydrophilic group, or a fluorine-containing hydrophobic group according to claim 1, and, further, which can be chemically bonded to hydroxy groups on the surface of a metal medical device.

Among the above silane compounds, the silane compound containing a polyoxyalkylene group may be any one which contains a polyoxyalkylene group and a group reactive with hydroxy groups on the surface of a metal medical device within the molecule. Such a silane compound can be chemically bonded to the surface of a metal medical device via the group reactive with hydroxy groups on the surface of the metal medical device. Further, the polyoxyalkylene group can impart lubricity and low protein adsorption properties and/or low cell adsorption properties to the surface of the metal medical device. Therefore, the effects of the present invention can be sufficiently achieved. Thus, in another suitable embodiment of the present invention, the silane compound contains a polyoxyalkylene group according to claim 1.

The silane compound containing a polyoxyalkylene group is preferably a silane compound that contains a hydrolyzable group and a polyoxyalkylene group (within one molecule). Examples of the hydrolyzable group include later-described hydrolyzable groups as typified by methoxy and ethoxy groups. Examples of the polyoxyalkylene group include later-described divalent groups having a linear or branched polyalkylene ether structure.

The silane compound containing a polyoxyalkylene group is represented by the following formula (1) or (2): 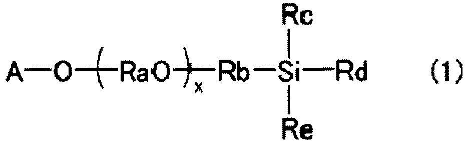 wherein A represents a hydrogen atom, a linear or branched C1-C3 alkyl group, or a linear or branched C1-C3 acyl group; Ra represents a linear or branched C1-C3 alkylene group; Rb represents a linear or branched C1-C5 alkylene group; Rc, Rd, and Re are the same as or different from one another, and each represent a halogen atom, a hydroxy group, a linear or branched C1-C3 alkyl group, or a linear or branched C1-C3 alkoxy group, provided that at least one of Rc, Rd, and Re is a halogen atom, a hydroxy group, or a linear or branched C1-C3 alkoxy group; and x represents the number of oxyalkylene repeating units each represented by RaO and is an integer of 2 to 500, 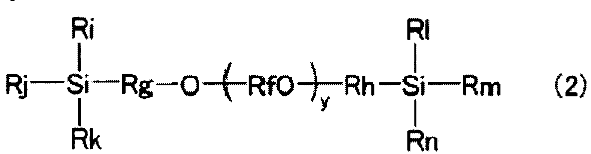 wherein Rf represents a linear or branched C1-C3 alkylene group; Rg and Rh are the same as or different from one another, and each represent a linear or branched C1-C5 alkylene group; Ri, Rj, Rk, Rl, Rm, and Rn are the same as or different from one another, and each represent a halogen atom, a hydroxy group, a linear or branched C1-C3 alkyl group, or a linear or branched C1-C3 alkoxy group, provided that at least one of Ri, Rj, Rk, Rl, Rm, and Rn is a halogen atom, a hydroxy group, or a linear or branched C1-C3 alkoxy group; and y represents the number of oxyalkylene repeating units each represented by RfO and is an integer of 2 to 500.

In view of affinity with water, A in the formula (1) is preferably a hydrogen atom, a linear or branched C1-C3 alkyl group, or a linear or branched C1-C3 acyl group, and more preferably a hydrogen atom, a linear or branched C1-C2 alkyl group, or a linear or branched C1-C2 acyl group. Specifically, A is preferably a hydrogen atom, a methyl group, an ethyl group, a propyl group, an isopropyl group, an acetyl group, or a propionyl group, and particularly preferably a hydrogen atom, a methyl group, an ethyl group, or an acetyl group.

In view of affinity with water, Ra in the formula (1) is preferably a linear or branched C1-C3 alkylene group. Specifically, it is preferably a methylene, ethylene, propylene, or isopropylene group, more preferably an ethylene, propylene, or isopropylene group, and particularly preferably an ethylene or propylene group.

In view of affinity with water, Rb in the formula (1) is preferably a linear or branched C1-C5 alkylene group, and more preferably a linear or branched C1-C3 alkylene group. Specifically, it is preferably a methylene, ethylene, propylene, isopropylene, or butylene group, and particularly preferably an ethylene, propylene, or isopropylene group.

Rc, Rd, and Re in the formula (1) are the same as or different from one another, and each represent a halogen atom, a hydroxy group, a linear or branched C1-C3 alkyl group, or a linear or branched C1-C3 alkoxy group, but preferably in view of affinity with water, at least one of Rc, Rd, and Re is a halogen atom, a hydroxy group, or a linear or branched C1-C3 alkoxy group. In a more preferred embodiment, Rc, Rd, and Re are the same as or different from one another and are each a fluorine atom, a chlorine atom, a bromine atom, a methyl group, an ethyl group, a propyl group, an isopropyl group, a methoxy group, an ethoxy group, a propoxy group, or an isopropoxy group, but at least one of them is a fluorine atom, a chlorine atom, a bromine atom, a methoxy group, an ethoxy group, a propoxy group, or an isopropoxy group. In a still more preferred embodiment, Rc, Rd, and Re are the same as or different from one another and are each a methoxy, ethoxy, propoxy, or isopropoxy group, or at least one of Rc, Rd, and Re is a fluorine atom, a chlorine atom, a bromine atom, a methoxy group, an ethoxy group, a propoxy group, or an isopropoxy group and the others are each a methyl, ethyl, propyl, or isopropyl group. In a particularly preferred embodiment, Rc, Rd, and Re are the same as or different from one another and are each a methoxy, ethoxy, or propoxy group; or two of Rc, Rd, and Re are the same as or different from one another and are each a methoxy, ethoxy, or propoxy group and the other one is a methyl, ethyl, propyl, or isopropyl group; or one of Rc, Rd, and Re is a fluorine, chlorine, or bromine atom and the other two are the same as or different from one another and are each a methyl, ethyl, propyl, or isopropyl group.

In the formula (1), x represents the number of oxyalkylene repeating units each represented by RaO and in view of affinity with water, it is preferably an integer of 2 to 500, more preferably an integer of 4 or more, still more preferably an integer of 5 or more. Also, it is more preferably an integer of 200 or less.

As described above, in the formula (1), (RaO)ₓ represents a divalent group having a linear or branched polyalkylene ether structure, and at least one of Rc, Rd, and Re is a hydrolyzable group.

In view of molecular rotational mobility or flexibility, Rf in the formula (2) is preferably a linear or branched C1-C3 alkylene group. Specifically, it is preferably a methylene, ethylene, propylene, or isopropylene group, and particularly preferably an ethylene, propylene, or isopropylene group.

Preferably, in view of hydrophilicity or hydrolyzability, Rg and Rh in the formula (2) are the same as or different from one another and are each a linear or branched C1-C5 alkylene group. More preferably, Rg and Rh are the same as or different from one another and are each a linear or branched C1-C3 alkylene group. Specifically, preferably Rg and Rh are the same as or different from one another and are each a methylene, ethylene, propylene, or isopropylene group, particularly preferably a methylene, ethylene, or propylene group.

Ri, Rj, Rk, Rl, Rm, and Rn in the formula (2) are the same as or different from one another, and each represent a halogen atom, a hydroxy group, a linear or branched C1-C3 alkyl group, or a linear or branched C1-C3 alkoxy group, but preferably in view of hydrophilicity or hydrolyzability, at least one of Ri, Rj, Rk, Rl, Rm, and Rn is a halogen atom, a hydroxy group, or a linear or branched C1-C3 alkoxy group. In more preferred embodiment, Ri, Rj, Rk, Rl, Rm, and Rn are the same as or different from one another and are each a fluorine atom, a chlorine atom, a bromine atom, a methyl group, an ethyl group, a propyl group, an isopropyl group, a methoxy group, an ethoxy group, a propoxy group, or an isopropoxy group, but at least one of them is a fluorine atom, a chlorine atom, a bromine atom, a methoxy group, an ethoxy group, a propoxy group, or an isopropoxy group. In a still more preferred embodiment, Ri, Rj, Rk, Rl, Rm, and Rn are the same as or different from one another and are each a methoxy, ethoxy, propoxy, or isopropoxy group; or at least one of Ri, Rj, Rk, Rl, Rm, and Rn is a fluorine atom, a chlorine atom, a bromine atom, a methoxy group, an ethoxy group, a propoxy group, or an isopropoxy group and the others are each a methyl, ethyl, propyl, or isopropyl group. In a particularly preferred embodiment, Ri, Rj, Rk, Rl, Rm, and Rn are the same as or different from one another and are each a methoxy, ethoxy, or propoxy group; or two of Ri, Rj and Rk, and two of Rl, Rm and Rn are the same as or different from one another and are each a methoxy, ethoxy, or propoxy group, and the other one out of the set of Ri, Rj and Rk and the other one out of the set of Rl, Rm and Rn are the same as or different from one another and are each a methyl, ethyl, propyl, or isopropyl group; or one of Ri, Rj and Rk, and one of Rl, Rm and Rn are the same as or different from one another and are each a fluorine, chlorine, or bromine atom, and the other two out of the set of Ri, Rj and Rk and the other two out of the set of Rl, Rm and Rn are the same as or different from one another and are each a methyl, ethyl, propyl, or isopropyl group.

In the formula (2), y represents the number of oxyalkylene repeating units each represented by RfO, and in view of molecular rotational mobility or flexibility, it is preferably an integer of 2 to 500, more preferably an integer of 4 or more, still more preferably an integer of 5 or more. Also, it is more preferably an integer of 200 or less.

As described above, in the formula (2), (RfO)_{y} represents a divalent group having a linear or branched polyalkylene ether structure, and at least one of Ri, Rj, Rk, Rl, Rm, and Rn is a hydrolyzable group.

Preferred examples of the compound represented by the formula (1) include compounds represented by the following formulas (3) to (5).

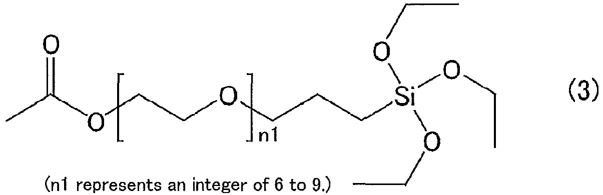

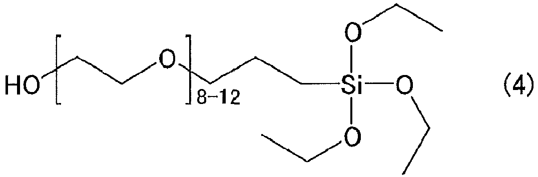

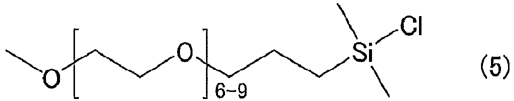

Preferred examples of the compound represented by the formula (2) include compounds represented by the following formulas (6) and (7).

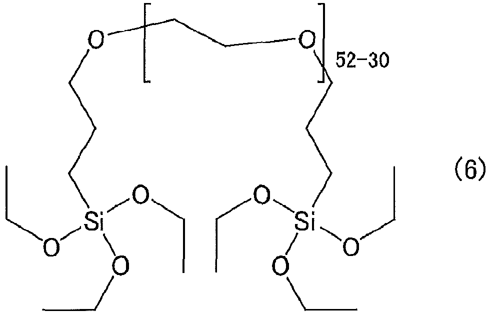

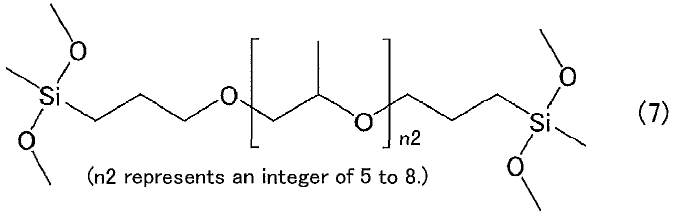

Examples of commercial products of the silane compound containing a polyoxyalkylene group include SIA0078.0 (a compound represented by the formula (3)), SIH6188.0 (a compound represented by the formula (4)), SIM6492.57 (a compound represented by the formula (5)), SIB1824.84 (a compound represented by the formula (6)), and SIB1660.0 (a compound represented by the formula (7)), all available from Gelest.

Among the silane compounds described earlier, the silane compound containing a metal salt-containing hydrophilic group may be any one which contains a metal salt-containing hydrophilic group and a group reactive with hydroxy groups on the surface of a metal medical device within the molecule. Such a silane compound can be chemically bonded to the surface of a metal medical device via the group reactive with hydroxy groups on the surface of the metal medical device. Further, the metal salt-containing hydrophilic group can impart lubricity and low protein adsorption properties and/or low cell adsorption properties to the surface of the metal medical device. Therefore, the effects of the present invention can be sufficiently achieved. Thus, in another suitable embodiment of the present invention, the silane compound contains a metal salt-containin hydrophilic group according to claim 1.

The silane compound containing a metal salt-containing hydrophilic group may contain one or two or more metal salt-containing hydrophilic groups within the molecule.

The silane compound containing a metal salt-containing hydrophilic group is preferably a silane compound that contains a hydrolyzable group and a metal salt-containing hydrophilic group (within one molecule). Examples of the hydrolyzable group include later-described hydrolyzable groups as typified by methoxy and ethoxy groups. Examples of the metal salt-containing hydrophilic group are described below.

The metal salt-containing hydrophilic group preferably includes a carboxylic acid metal salt, a phosphonic acid ester metal salt, or a sulfonic acid metal salt. Such a metal salt-containing hydrophilic group improves water-holding properties.

The metal of the metal salt-containing hydrophilic group is preferably an alkali metal or an alkaline earth metal. When the metal of the metal salt-containing hydrophilic group is an alkali metal or an alkaline earth metal, water-holding properties are improved. The metal is particularly preferably lithium, sodium, potassium, magnesium, or calcium.

Thus, in another suitable embodiment of the present invention, the metal salt-containing hydrophilic group is an alkali metal salt-containing hydrophilic group or an alkaline earth metal salt-containing hydrophilic group according to claim 1.

Examples of the group reactive with hydroxy groups on the surface of a metal medical device, in the silane compound containing a metal salt-containing hydrophilic group, include hydrolyzable group-containing alkoxysilyl groups such as a methoxysilyl group and an ethoxysilyl group; and hydroxysilyl groups. The silane compound containing a metal salt-containing hydrophilic group may contain one or two or more groups reactive with hydroxy groups on the surface of a metal medical device within the molecule.

In particular, the silane compound containing a metal salt-containing hydrophilic group preferably contains a trimethoxysilyl, triethoxysilyl, or trihydroxysilyl group as the group reactive with hydroxy groups on the surface of a metal medical device.

The silane compound containing a metal salt-containing hydrophilic group is a silane compound represented by the following formula (8) or (9): 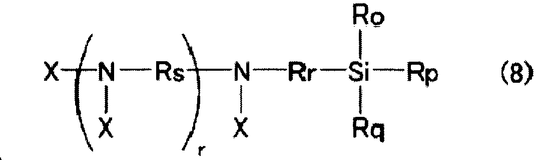 wherein Ro, Rp, and Rq are the same as or different from one another, and each represent a halogen atom, a hydroxy group, a linear or branched C1-C3 alkyl group, or a linear or branched C1-C3 alkoxy group, provided that at least one of Ro, Rp, and Rq is a halogen atom, a hydroxy group, or a linear or branched C1-C3 alkoxy group; Rr and Rs are the same as or different from one another, and each represent a linear or branched C1-C7 alkylene group; each X is the same or different and represents a metal salt-containing hydrophilic group; and r represents 0 or 1, 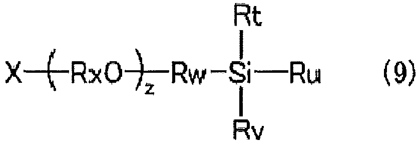 wherein Rt, Ru, and Rv are the same as or different from one another, and each represent a halogen atom, a hydroxy group, a linear or branched C1-C3 alkyl group, or a linear or branched C1-C3 alkoxy group, provided that at least one of Rt, Ru, and Rv is a halogen atom, a hydroxy group, or a linear or branched C1-C3 alkoxy group; Rw represents a linear or branched C1-C5 alkylene group; Rx represents a linear or branched C1-C3 alkylene group; each X is the same or different and represents a metal salt-containing hydrophilic group; and z represents the number of oxyalkylene repeating units each represented by RxO and is an integer of 2 to 500.

Ro, Rp, and Rq in the formula (8) are the same as or different from one another, and each represent a halogen atom, a hydroxy group, a linear or branched C1-C3 alkyl group, or a linear or branched C1-C3 alkoxy group, but preferably in view of affinity with water, at least one of Ro, Rp, and Rq is a halogen atom, a hydroxy group, or a linear or branched C1-C3 alkoxy group. In a more preferred embodiment, Ro, Rp, and Rq are the same as or different from one another and are each a fluorine atom, a chlorine atom, a bromine atom, a hydroxy group, a methyl group, an ethyl group, a propyl group, an isopropyl group, a methoxy group, an ethoxy group, a propoxy group, or an isopropoxy group, but at least one of them is a fluorine atom, a chlorine atom, a bromine atom, a hydroxy group, a methoxy group, an ethoxy group, a propoxy group, or an isopropoxy group. In a still more preferred embodiment, Ro, Rp, and Rq are the same as or different from one another and are each a hydroxy, methoxy, ethoxy, propoxy, or isopropoxy group; or at least one of Ro, Rp, and Rq is a fluorine atom, a chlorine atom, a bromine atom, a hydroxy group, a methoxy group, an ethoxy group, a propoxy group, or an isopropoxy group and the others are each a methyl, ethyl, propyl, or isopropyl group. In a particularly preferred embodiment, Ro, Rp, and Rq are the same as or different from one another and are each a hydroxy, methoxy, ethoxy, or propoxy group.

Preferably, in view of affinity with water, Rr and Rs in the formula (8) are the same as or different from one another and are each a linear or branched C1-C7 alkylene group, more preferably a linear or branched C1-C5 alkylene group, still more preferably a linear or branched C1-C4 alkylene group, and particularly preferably a linear or branched C1-C3 alkylene group. Specifically, they are each preferably a methylene, ethylene, propylene, isopropylene, butylene, or heptene group, and more preferably an ethylene, propylene, isopropylene, or butylene group.

Preferred examples of the metal salt-containing hydrophilic group designated by X in the formula (8) include functional groups represented by the formulas (10) to (12) below. Among these, the functional group represented by the formula (12) is particularly preferred in view of lubricity and sliding properties.

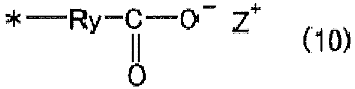

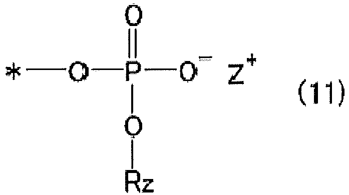

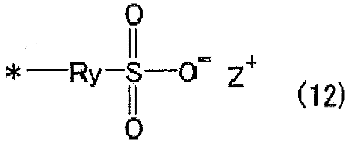

Ry in the formulas (10) and (12) represents a linear or branched C1-C5 alkylene group. Rz in the formula (11) represents a linear or branched C1-C3 alkyl group. In the formulas (10) to (12), Z⁺ represents a monovalent metal ion, and particularly preferably a sodium ion, a potassium ion, or a lithium ion. Also in the formulas (10) to (12), * represents a bond.

In particular, X in the formula (8) is more preferably a functional group represented by the formula (10) in which Ry is a methylene, ethylene, or propylene group and Z⁺ is a sodium or potassium ion; a functional group represented by the formula (11) in which Rz is a methyl, ethyl, or propyl group and Z⁺ is a sodium or potassium ion; or a functional group represented by the formula (12) in which Ry is a methylene, ethylene, or propylene group and Z⁺ is a sodium or potassium ion.

Rt, Ru, and Rv in the formula (9) are the same as or different from one another, and each represent a halogen atom, a hydroxy group, a linear or branched C1-C3 alkyl group, or a linear or branched C1-C3 alkoxy group, but preferably in view of affinity with water, at least one of Rt, Ru, and Rv is a halogen atom, a hydroxy group, or a linear or branched C1-C3 alkoxy group. In a more preferred embodiment, Rt, Ru, and Rv are the same as or different from one another and are each a fluorine atom, a chlorine atom, a bromine atom, a hydroxy group, a methyl group, an ethyl group, a propyl group, an isopropyl group, a methoxy group, an ethoxy group, a propoxy group, or an isopropoxy group, but at least one of them is a fluorine atom, a chlorine atom, a bromine atom, a hydroxy group, a methoxy group, an ethoxy group, a propoxy group, or an isopropoxy group. In a still more preferred embodiment, Rt, Ru, and Rv are the same as or different from one another and are each a hydroxy, methoxy, ethoxy, propoxy, or isopropoxy group; or at least one of Rt, Ru, and Rv is a fluorine atom, a chlorine atom, a bromine atom, a hydroxy group, a methoxy group, an ethoxy group, a propoxy group, or an isopropoxy group and the others are each a methyl, ethyl, propyl, or isopropyl group. In a particularly preferred embodiment, Rt, Ru, and Rv are the same as or different from one another and are each a hydroxy, methoxy, ethoxy, or propoxy group.

In view of affinity with water, Rw in the formula (9) is preferably a linear or branched C1-C5 alkylene group, and more preferably a linear or branched C1-C4 alkylene group. Specifically, it is preferably a methylene, ethylene, propylene, isopropylene, or butylene group, more preferably a methylene, ethylene, propylene, or butylene group.

In view of affinity with water, Rx in the formula (9) is preferably a linear or branched C1-C3 alkylene group. Specifically, it is preferably a methylene, ethylene, propylene, or isopropylene group, and particularly preferably an ethylene, propylene, or isopropylene group.

X in the formula (9) is as defined for X in the formula (8).

In the formula (9), z represents the number of oxyalkylene repeating units each represented by RxO, and in view of affinity with water, it is preferably an integer of 0 to 500. When the oxyalkylene group represented by RxO is contained, z is more preferably an integer of 2 to 500, still more preferably an integer of 5 or more, and particularly preferably an integer of 7 or more. Also, it is still more preferably an integer of 200 or less.

As described above, X in the formula (8) or (9) represents a metal salt-containing hydrophilic group, and at least one of Ro, Rp, and Rq in the formula (8) or at least one of Rt, Ru, and Rv in the formula (9) is a hydrolyzable group.

Preferred examples of the silane compound containing a metal salt-containing hydrophilic group include compounds represented by the following formulas (13) to (15).

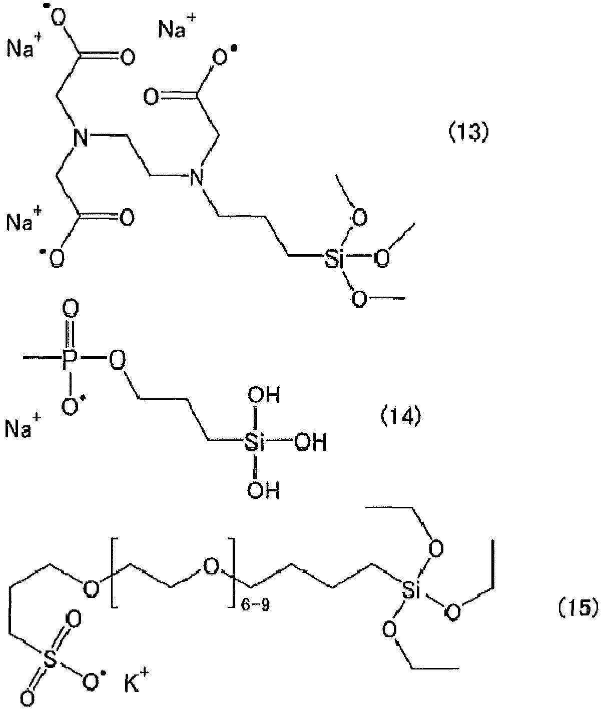

Examples of commercial products of the silane compound containing a metal salt-containing hydrophilic group include SIT8402.0 (a compound represented by the formula (13)), SIT8378.5 (a compound represented by the formula (14)), and SIT8192.2 (a compound represented by the formula (15)), all available from Gelest.

Among the silane compounds described earlier, the silane compound containing a halogen salt-containing hydrophilic group may be any one which contains a halogen salt-containing hydrophilic group and a group reactive with hydroxy groups on the surface of a metal medical device within the molecule. Such a silane compound, as defined in claim 1, can be chemically bonded to the surface of a metal medical device via the group reactive with hydroxy groups on the surface of the metal medical device. Further, the halogen salt-containing hydrophilic group can impart lubricity and low protein adsorption properties and/or low cell adsorption properties to the surface of the metal medical device. Therefore, the effects of the present invention can be sufficiently achieved. Thus, in another suitable embodiment of the present invention the silane compound contains a halogen salt-containing hydrophilic group according to claim 1.

The silane compound containing a halogen salt-containing hydrophilic group may contain one or two or more halogen salt-containing hydrophilic groups within the molecule.

In particular, the silane compound containing a halogen salt-containing hydrophilic group is preferably a silane compound that contains a hydrolyzable group and a halogen salt-containing hydrophilic group (within one molecule). Examples of the hydrolyzable group include later-described hydrolyzable groups as typified by methoxy and ethoxy groups. Examples of the halogen salt-containing hydrophilic group are described below.

The halogen salt-containing hydrophilic group preferably includes an ammonium halogen salt. Such a halogen salt-containing hydrophilic group improves water-holding properties.

The halogen of the halogen salt-containing hydrophilic group may be fluorine, chlorine, bromine, or iodine. Among these, in view of water-holding properties and stability, it is preferably chlorine or bromine, particularly preferably chlorine. Thus, in another suitable embodiment of the present invention, the halogen salt-containing hydrophilic group is a chlorine salt-containing hydrophilic group.

Examples of the group reactive with hydroxy groups on the surface of a metal medical device, in the silane compound containing a halogen salt-containing hydrophilic group, include hydrolyzable group-containing alkoxysilyl groups such as a methoxysilyl group and an ethoxysilyl group; and hydroxysilyl groups. The silane compound containing a halogen salt-containing hydrophilic group may contain one or two or more groups reactive with hydroxy groups on the surface of a metal medical device within the molecule.

In particular, the silane compound containing a halogen salt-containing hydrophilic group preferably contains a trimethoxysilyl, triethoxysilyl, or trihydroxysilyl group, particularly preferably a trimethoxysilyl group, as the group reactive with hydroxy groups on the surface of a metal medical device.

The silane compound containing a halogen salt-containing hydrophilic group is a silane compound represented by the formula (16) below or a silane compound containing a linking unit A represented by the formula (17) below and a linking unit B represented by the formula (18) below. Among these, in view of lubricity and sliding properties, it is particularly preferably a silane compound containing a linking unit A represented by the formula (17) and a linking unit B represented by the formula (18).

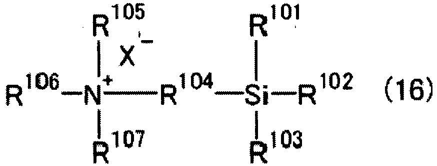

In the formula (16), R¹⁰¹, R¹⁰², and R¹⁰³ are the same as or different from one another, and each represent a halogen atom, a hydroxy group, a linear or branched C1-C3 alkyl group, or a linear or branched C1-C3 alkoxy group, provided that at least one of R¹⁰¹, R¹⁰², and R¹⁰³ is a halogen atom, a hydroxy group, or a linear or branched C1-C3 alkoxy group; R¹⁰⁴ represents a linear or branched C1-C7 alkylene group; R¹⁰⁵, R¹⁰⁶, and R¹⁰⁷ are the same as or different from one another, and each represent a hydrogen atom or a linear or branched C1-C5 alkyl group optionally containing an ether linkage; and X' represents a halogen atom.

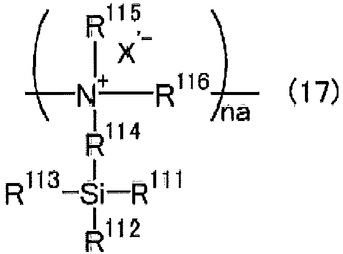

In the formula (17), R¹¹¹, R¹¹², and R¹¹³ are the same as or different from one another, and each represent a halogen atom, a hydroxy group, a linear or branched C1-C3 alkyl group, or a linear or branched C1-C3 alkoxy group, provided that at least one of R¹¹¹, R¹¹², and R¹¹³ is a halogen atom, a hydroxy group, or a linear or branched C1-C3 alkoxy group; R¹¹⁴ represents a linear or branched C1-C7 alkylene group; R¹¹⁵ represents a hydrogen atom or a linear or branched C1-C5 alkyl group optionally containing an ether linkage; R¹¹⁶ represents a linear or branched C1-C5 alkylene group; X' represents a halogen atom; and na represents an integer of 1 or more.

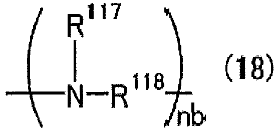

In the formula (18), R¹¹⁷ represents a hydrogen atom or a linear or branched C1-C5 alkyl group optionally containing an ether linkage; R¹¹⁸ represents a linear or branched C1-C5 alkylene group; and nb represents an integer of 1 or more.

R¹⁰¹, R¹⁰², and R¹⁰³ in the formula (16) are the same as or different from one another, and each represent a halogen atom, a hydroxy group, a linear or branched C1-C3 alkyl group, or a linear or branched C1-C3 alkoxy group, but preferably in view of affinity with water, at least one of R¹⁰¹, R¹⁰², and R¹⁰³ is a halogen atom, a hydroxy group, or a linear or branched C1-C3 alkoxy group. In a more preferred embodiment, R¹⁰¹, R¹⁰², and R¹⁰³ are the same as or different from one another and are each a fluorine atom, a chlorine atom, a bromine atom, a hydroxy group, a methyl group, an ethyl group, a propyl group, an isopropyl group, a methoxy group, an ethoxy group, a propoxy group, or an isopropoxy group, but at least one of them is a fluorine atom, a chlorine atom, a bromine atom, a hydroxy group, a methoxy group, an ethoxy group, a propoxy group, or an isopropoxy group. In a still more preferred embodiment, R¹⁰¹, R¹⁰², and R¹⁰³ are the same as or different from one another and are each a hydroxy, methoxy, ethoxy, propoxy, or isopropoxy group; or at least one of R¹⁰¹, R¹⁰², and R¹⁰³ is a fluorine atom, a chlorine atom, a bromine atom, a hydroxy group, a methoxy group, an ethoxy group, a propoxy group, or an isopropoxy group and the others are each a methyl, ethyl, propyl, or isopropyl group. In a particularly preferred embodiment, R¹⁰¹, R¹⁰², and R¹⁰³ are the same as or different from one another and are each a methoxy, ethoxy, or propoxy group.

In view of affinity with water, R¹⁰⁴ in the formula (16) is preferably a linear or branched C1-C7 alkylene group, more preferably a linear or branched C1-C5 alkylene group, still more preferably a linear or branched C1-C4 alkylene group, and particularly preferably a linear or branched C1-C3 alkylene group. Specifically, it is preferably a methylene, ethylene, propylene, isopropylene, butylene, or heptene group, more preferably an ethylene, propylene, isopropylene, or butylene group.

Preferably, in view of affinity with water, R¹⁰⁵, R¹⁰⁶, and R¹⁰⁷ in the formula (16) are the same as or different from one another and are each a hydrogen atom or a linear or branched C1-C5 alkyl group optionally containing an ether linkage. When the linear or branched C1-C5 alkyl group contains an ether linkage, the ether linkage may be incorporated at any position of the alkyl group, and one or two or more ether linkages may be incorporated per alkyl group. More preferably, R¹⁰⁵, R¹⁰⁶, and R¹⁰⁷ are the same as or different from one another and are each a hydrogen atom, a linear or branched C1-C5 alkyl group, or a linear or branched C1-C5 alkoxyalkyl group. In a still more preferred embodiment, at least one of R¹⁰⁵, R¹⁰⁶, and R¹⁰⁷ is a linear or branched C1-C5 alkyl group or a linear or branched C1-C5 alkoxyalkyl group and the others are each a hydrogen atom, a linear or branched C1-C5 alkyl group, or a linear or branched C1-C5 alkoxyalkyl group. In a particularly preferred embodiment, two of R¹⁰⁵, R¹⁰⁶, and R¹⁰⁷ are each a linear or branched C1-C5 alkoxyalkyl group and the other one is a hydrogen atom; or R¹⁰⁵, R¹⁰⁶, and R¹⁰⁷ are the same as or different from one another and are each a linear or branched C1-C5 alkyl group.

Specific examples of the linear or branched C1-C5 alkyl group include methyl, ethyl, propyl, isopropyl, n-butyl, isobutyl, t-butyl, and pentyl groups. Preferred are methyl, ethyl, propyl, isopropyl, n-butyl, isobutyl, and t-butyl groups, more preferred are methyl, ethyl, propyl, isopropyl, isobutyl, and t-butyl groups, and particularly preferred are methyl, ethyl, and isobutyl groups.

Specific examples of the linear or branched C1-C5 alkoxyalkyl group include methoxymethyl, methoxyethyl, methoxypropyl, methoxyisopropyl, ethoxymethyl, ethoxyethyl, ethoxypropyl, ethoxyisopropyl, propoxyethyl, and propoxyethyl groups. Preferred are methoxymethyl, methoxyethyl, methoxypropyl, methoxyisopropyl, ethoxymethyl, ethoxyethyl, and ethoxypropyl groups; more preferred are methoxymethyl, methoxyethyl, methoxypropyl, and methoxyisopropyl groups; and particularly preferred are methoxymethyl and methoxyethyl groups.

X' in the formula (16) represents a halogen atom, and specifically may be a fluorine, chlorine, bromine, or iodine atom. Among these, in view of water-holding properties, it is preferably a chlorine or bromine atom, and particularly preferably a chlorine atom.

R¹¹¹, R¹¹², and R¹¹³ in the formula (17) are the same as or different from one another, and each represent a halogen atom, a hydroxy group, a linear or branched C1-C3 alkyl group, or a linear or branched C1-C3 alkoxy group, but preferably in view of affinity with water, at least one of R¹¹¹, R¹¹², and R¹¹³ is a halogen atom, a hydroxy group, or a linear or branched C1-C3 alkoxy group. In a more preferred embodiment, R¹¹¹, R¹¹², and R¹¹³ are the same as or different from one another and are each a fluorine atom, a chlorine atom, a bromine atom, a hydroxy group, a methyl group, an ethyl group, a propyl group, an isopropyl group, a methoxy group, an ethoxy group, a propoxy group, or an isopropoxy group, but at least one of them is a fluorine atom, a chlorine atom, a bromine atom, a hydroxy group, a methoxy group, an ethoxy group, a propoxy group, or an isopropoxy group. In a still more preferred embodiment, R¹¹¹, R¹¹², and R¹¹³ are the same as or different from one another and are each a hydroxy, methoxy, ethoxy, propoxy, or isopropoxy group; or at least one of R¹¹¹, R¹¹², and R¹¹³ is a fluorine atom, a chlorine atom, a bromine atom, a hydroxy group, a methoxy group, an ethoxy group, a propoxy group, or an isopropoxy group and the others are each a methyl, ethyl, propyl, or isopropyl group. In a particularly preferred embodiment, R¹¹¹, R¹¹², and R¹¹³ are the same as or different from one another and are each a methoxy, ethoxy, or propoxy group.

In view of affinity with water, R¹¹⁴ in the formula (17) is preferably a linear or branched C1-C7 alkylene group, more preferably a linear or branched C1-C5 alkylene group, still more preferably a linear or branched C1-C4 alkylene group, and particularly preferably a linear or branched C1-C3 alkylene group. Specifically, it is preferably a methylene, ethylene, propylene, isopropylene, butylene, or heptene group, more preferably an ethylene, propylene, isopropylene, or butylene group.

In view of affinity with water, R¹¹⁵ in the formula (17) is preferably a hydrogen atom or a linear or branched C1-C5 alkyl group optionally containing an ether linkage. When the linear or branched C1-C5 alkyl group contains an ether linkage, the ether linkage may be incorporated at any position of the alkyl group, and one or two or more ether linkages may be incorporated per alkyl group. R¹¹⁵ is more preferably a hydrogen atom, a linear or branched C1-C5 alkyl group, or a linear or branched C1-C5 alkoxyalkyl group, and still more preferably a hydrogen atom.

The linear or branched C1-C5 alkyl group and the linear or branched C1-C5 alkoxyalkyl group are as described above.

In view of affinity with water, R¹¹⁶ in the formula (17) is preferably a linear or branched C1-C5 alkylene group, more preferably a linear or branched C1-C4 alkylene group, and still more preferably a linear or branched C1-C3 alkylene group. Specifically, it is preferably a methylene, ethylene, propylene, isopropylene, or butylene group, more preferably a methylene, ethylene, propylene, or butylene group, and particularly preferably a methylene, ethylene, or propylene group.

X' in the formula (17) is as defined for X' in the formula (16).

In the formula (17), na represents the number of repeating linking units A and is an integer of 1 or more. na is preferably an integer of 2 or more, more preferably an integer of 3 or more, while it is preferably an integer of 500 or less, more preferably an integer of 100 or less, still more preferably an integer of 50 or less.

R¹¹⁷ in the formula (18) is as defined for R¹¹⁵ in the formula (17).

R¹¹⁸ in the formula (18) is as defined for R¹¹⁶ in the formula (17).

In the formula (18), nb represents the number of repeating linking units B and is an integer of 1 or more. nb is preferably an integer of 2 or more, more preferably an integer of 3 or more, while it is preferably an integer of 500 or less, more preferably an integer of 100 or less, still more preferably an integer of 50 or less.

In the silane compound containing a linking unit A represented by the formula (17) and a linking unit B represented by the formula (18), the sum (na + nb) of the number of repeating linking units A (na) and the number of repeating linking units B (nb) is preferably in the range of 2 to 1,000, more preferably of 5 to 200.

In the silane compound containing a linking unit A represented by the formula (17) and a linking unit B represented by the formula (18), the linking unit A content is preferably 5 mol% or more, more preferably 10 mol% or more, while it is preferably 75 mol% or less, more preferably 50 mol% or less, still more preferably 40 mol% or less. On the other hand, the linking unit B content is preferably 25 mol% or more, more preferably 50 mol% or more, still more preferably 60 mol% or more, while it is preferably 95 mol% or less, more preferably 90 mol% or less. When the linking unit A content and the linking unit B content fall within the respective ranges described above, the effects of the present invention can be more suitably achieved.

The linking unit A or B content refers to the amount including the linking unit A or B, if any, located at the end of the silane compound. The form of the linking unit A or B located at the end of the silane compound is not particularly limited, as long as it forms a unit corresponding to the formula (17) or (18) representing the linking unit A or B.

The silane compound containing a linking unit A represented by the formula (17) and a linking unit B represented by the formula (18) may contain other linking units as long as it contains the linking units A and B. The combined content of the linking units A and B is preferably 95 mol% or more, more preferably 98 mol% or more, particularly preferably 100 mol%.

In the silane compound containing a linking unit A represented by the formula (17) and a linking unit B represented by the formula (18), the linking units may be joined in any order and may be arranged randomly, in blocks, or in an alternating sequence.

The silane compound containing a linking unit A represented by the formula (17) and a linking unit B represented by the formula (18) preferably has a weight average molecular weight of 1,000 to 10,000. When the weight average molecular weight of the silane compound is in the above range, the effects of the present invention can be more suitably achieved.

The weight average molecular weight can be measured using a gel permeation chromatograph (GPC), calibrated with polystyrene standards.

Thus, in the silane compound represented by the formula (16) or the silane compound containing a linking unit A represented by the formula (17) and a linking unit B represented by the formula (18), X' represents a halogen atom, and at least one of R¹⁰¹, R¹⁰², and R¹⁰³ in the formula (16), or at least one of R¹¹¹, R¹¹², and R¹¹³ in the formula (17) is a hydrolyzable group.

Preferred examples of the silane compound containing a halogen salt-containing hydrophilic group include compounds represented by the following formulas (19) and (20) and compounds containing a linking unit represented by the following formula (21) and a linking unit represented by the following formula (22).

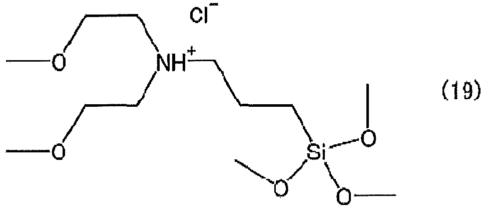

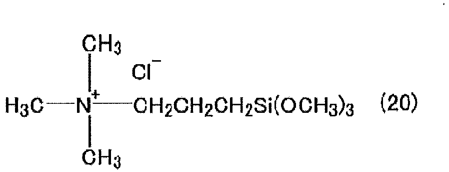

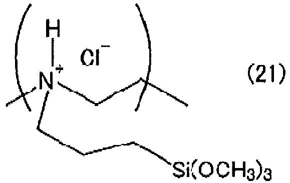

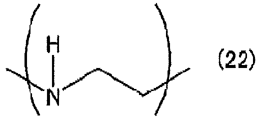

Examples of commercial products of the silane compound containing a halogen salt-containing hydrophilic group include SIB1500.0 (a compound represented by the formula (19)), SIT8415.0 (a compound represented by the formula (20)), and SSP-060 (a compound containing a linking unit represented by the formula (21) and a linking unit represented by the formula (22)), all available from Gelest.

Among the silane compounds described earlier, the silane compound containing a fluorine-containing hydrophobic group may be any one which contains a fluorine atom-containing hydrophobic group and a group reactive with hydroxy groups on the surface of a metal medical device within the molecule. Such a silane compound can be chemically bonded to the surface of a metal medical device via the group reactive with hydroxy groups on the surface of the metal medical device. Further, the fluorine-containing hydrophobic group can impart lubricity and low protein adsorption properties and/or low cell adsorption properties to the surface of the metal medical device. Therefore, the effects of the present invention can be sufficiently achieved. Thus, in another suitable embodiment of the present invention, the silane compound contains a fluorine-containing hydrophobic group according to claim 1.

In particular, the silane compound containing a fluorine-containing hydrophobic group is preferably a silane compound having a perfluoroether structure, and specifically, for example, a silane compound that contains a hydrolyzable group and a perfluoropolyether group (within one molecule). Thus, in another suitable embodiment of the present invention, the fluorine-containing hydrophobic group is a perfluorooxyalkylene group according to claim 1.

Examples of the hydrolyzable group include later-described hydrolyzable groups as typified by methoxy and ethoxy groups. Examples of the perfluoroether group (perfluorooxyalkylene group) include later-described divalent groups having a linear perfluoropolyalkylene ether structure.

Moreover, the silane compound containing a fluorine-containing hydrophobic group preferably contains a fluoroalkyl group such as a perfluoroalkyl group.

The silane compound containing a fluorine-containing hydrophobic group is a silane compound represented by the following formula (23) or (24).

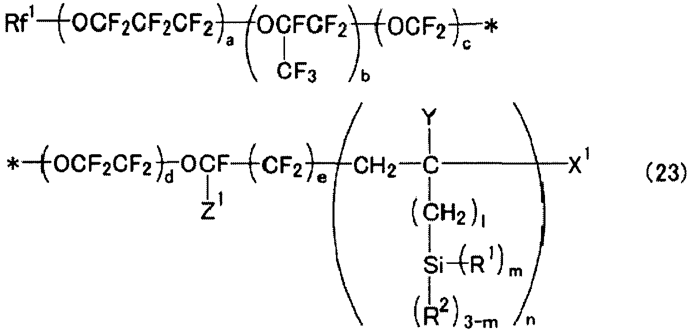

In the formula (23), Rf¹ represents a perfluoroalkyl group; Z¹ represents fluorine or a trifluoromethyl group; a, b, c, d, and e are the same as or different from one another, and each represent an integer of 0 or 1 or more, provided that a + b + c + d + e is 1 or more and the order of the repeating units parenthesized by subscripts a, b, c, d, and e occurring in the above formula is not limited to that shown; Y represents hydrogen or a C1-C4 alkyl group; X¹ represents hydrogen, bromine, or iodine; R¹ represents a hydroxy group or a hydrolyzable substituent; R² represents hydrogen or a monovalent hydrocarbon group; 1 represents 0, 1, or 2; m represents 1, 2, or 3; and n represents an integer of 1 or more. Please note that the two ends marked by * are directly bonded to each other.

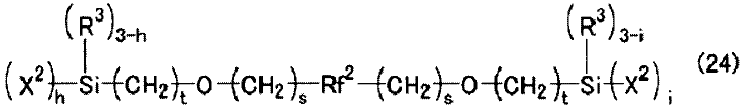

In the formula (24), Rf² represents a divalent group that contains a unit represented by -(CₖF₂ₖ)O- where k is an integer of 1 to 6, and has a non-branched linear perfluoropolyalkylene ether structure; each R³ is the same or different and represents a monovalent C1-C8 hydrocarbon group; each X² is the same or different and represents a hydrolyzable group or a halogen atom; each s is the same or different and represents an integer of 0 to 2; each t is the same or different and represents an integer of 1 to 5; and h and i are the same as or different from one another, and each represent 2 or 3.

Rf¹ in the formula (23) may be any perfluoroalkyl group that can be used in a common organic-containing fluoropolymer, and examples include linear or branched C1-C16 groups. Among these, it is preferably CF₃-, C₂F₅-, or C₃F₇-.

In the formula (23), a, b, c, d, and e each represent the number of repeating units in the perfluoropolyether chain which forms the backbone of the silane compound containing a fluorine-containing hydrophobic group, and are each independently preferably 0 to 200, more preferably 0 to 50. Moreover, a + b + c + d + e (sum of a to e) is preferably 1 to 100. The order of the repeating units parenthesized by subscripts a, b, c, d, and e occurring in the formula (23) is not limited to the order shown in the formula (23), and the repeating units may be joined in any order.

Examples of the C1-C4 alkyl group designated by Y in the formula (23) include methyl, ethyl, propyl, and butyl groups, and the C1-C4 alkyl group may be linear or branched. When X¹ is bromine or iodine, the silane compound containing a fluorine-containing hydrophobic group can easily be chemically bonded.

Preferred examples of the hydrolyzable substituent designated by R¹ in the formula (23) include, but are not limited to, halogens, -OR¹¹, -OCOR¹¹, -OC(R¹¹)=C(R¹²)₂, - ON=C(R¹¹)₂, and -ON=CR¹³, wherein R¹¹ represents an aliphatic hydrocarbon group or an aromatic hydrocarbon group, R¹² represents hydrogen or a C1-C4 aliphatic hydrocarbon group, and R¹³ represents a divalent C3-C6 aliphatic hydrocarbon group. More preferred are chlorine, -OCH₃, and -OC₂H₅.

Preferred examples of the monovalent hydrocarbon group designated by R² include, but are not limited to, methyl, ethyl, propyl, and butyl groups, and the monovalent hydrocarbon group may be linear or branched.

In the formula (23), 1 represents the number of carbon atoms of the alkylene group between the carbon in the perfluoropolyether chain and the silicon attached thereto and is preferably 0; and m represents the number of substituents R¹ bonded to the silicon to which R² is bonded through a bond not attached to R¹. The upper limit of n is not particularly limited, and is preferably an integer of 1 to 10.

In the formula (24), on the other hand, the Rf² group is preferably, but not limited to, such that when each s is 0, the ends of the Rf². group bonded to the oxygen atoms in the formula (24) are not oxygen atoms. Moreover, k in Rf² is preferably an integer of 1 to 4. Specific examples of the Rf² group include -CF₂CF₂O (CF₂CF₂CF₂O)ⱼCF₂CF₂- in which j is an integer of 1 or more, preferably of 1 to 50, more preferably of 10 to 40; and -CF₂(OC₂F₄)ₚ-(OCF₂)_{q}- in which p and q each represent an integer of 1 or more, preferably of 1 to 50, more preferably of 10 to 40, and the sum of p and q is an integer of 10 to 100, preferably of 20 to 90, more preferably of 40 to 80, and the repeating units (OC₂F₄) and (OCF₂) are randomly arranged.

R³ in the formula (24) is preferably a monovalent C1-C3 hydrocarbon group, and examples include alkyl groups such as methyl, ethyl, propyl, butyl, pentyl, hexyl, heptyl, and octyl groups; cycloalkyl groups such as cyclopentyl and cyclohexyl groups; aryl groups such as phenyl, tolyl, and xylyl groups; aralkyl groups such as benzyl and phenethyl groups; and alkenyl groups such as vinyl, allyl, butenyl, pentenyl, and hexenyl groups. Among these, a methyl group is preferred.

Examples of the hydrolyzable group designated by X² in the formula (24) include alkoxy groups such as methoxy, ethoxy, propoxy, and butoxy groups; alkoxyalkoxy groups such as methoxymethoxy, methoxyethoxy, and ethoxyethoxy groups; alkenyloxy groups such as allyloxy and isopropenoxy groups; acyloxy groups such as acetoxy, propionyloxy, butylcarbonyloxy, and benzoyloxy groups; ketoxime groups such as dimethylketoxime, methylethylketoxime, diethylketoxime, cyclopennoxime, and cyclohexanoxime groups; amino groups such as N-methylamino, N-ethylamino, N-propylamino, N-butylamino, N,N-dimethylamino, N,N-diethylamino, and N-cyclohexylamino groups; amide groups such as N-methylacetamide, N-ethylacetamide, and N-methylbenzamide groups; and aminooxy groups such as N,N-dimethylaminooxy and N,N-diethylaminooxy groups. Moreover, examples of the halogen atom designated by X² include chlorine, bromine, and iodine atoms. Among the above examples, methoxy, ethoxy, and isopropenoxy groups, and a chlorine atom are preferred.

In the formula (24), s is preferably 1; t is preferably 3; and in view of hydrolyzability, h and i are each preferably 3.

In view of continuous demoldability, the silane compound containing a fluorine-containing hydrophobic group preferably has a weight average molecular weight in the range of 1,000 to 10,000.

The weight average molecular weight can be measured using a gel permeation chromatograph (GPC), calibrated with polystyrene standards.

Examples of commercial products of the silane compound containing a fluorine-containing hydrophobic group include OPTOOL DSX available from Daikin Industries, Ltd. and KY-130 available from Shin-Etsu Chemical Co., Ltd.

Examples of metal medical devices that may be used in the present invention include guide wires, stents, needles, stylets, and metal tubes. Especially in the case of guide wires, the effects of imparting lubricity, of improving the durability of the lubricant layer on the surface, and of suppressing deterioration of sliding properties work most effectively, so that the insertability, pushability, and sliding properties in body cavities or tissues and the durability of these properties are improved. Consequently, the effects of the present invention can be particularly remarkably achieved. Thus, in another suitable embodiment of the present invention, the metal medical device of the present invention is a guide wire. Especially in the case of stents, on the other hand, the effects of imparting low protein adsorption properties and/or low cell adsorption properties, of improving the durability of the lubricant layer on the surface, and of suppressing deterioration of low protein adsorption properties and/or low cell adsorption properties work most effectively, so that the lubricant layer is prevented from being washed away and removed during the long indwelling time, and the effect of preventing adhesion of proteins or cells to the stent surface is maintained. Consequently, the effects of the present invention can be particularly remarkably achieved. Thus, in another suitable embodiment of the present invention, the metal medical device of the present invention is a stent.

Exemplary materials of the metal medical device of the present invention include metals such as stainless steel, nickel-titanium alloys, iron, titanium, aluminum, tin, and zinc-tungsten alloys. Among these, stainless steel and nickel-titanium alloys are preferred in view of bonding between the surface of the metal medical device and the lubricant layer. Thus, in another suitable embodiment of the present invention, the metal medical device of the present invention includes a core wire made of stainless steel or nickel-titanium alloys.

The metal medical device having lubricity and low protein adsorption properties and/or low cell adsorption properties of the present invention can be prepared, for example, by treating the surface of a metal medical device with the silane compound described above. The treatment of the surface of a metal medical device with the silane compound containing a functional group that is a polyoxyalkylene group, a metal salt-containing hydrophilic group, a halogen salt-containing hydrophilic group, or a fluorine-containing hydrophobic group causes hydrolysis of the silane compound, a dehydration or deoxygenation condensation reaction between the silane compound and hydroxy groups on the surface of the metal medical device, and the like, as a result of which the hydroxy groups on the surface of the metal medical device and the silane compound are chemically bonded to each other. Therefore, lubricity and low protein adsorption properties and/or low cell adsorption properties are imparted to the surface of the metal medical device. Further, the durability of the lubricant layer on the surface is improved to suppress deterioration of the sliding properties and low protein adsorption properties and/or low cell adsorption properties of the metal medical device having lubricity and low protein adsorption properties and/or low cell adsorption properties.

Thus, another aspect of the present invention relates to a method for preparing a metal medical device according to claim 2 and having lubricity and low protein adsorption properties and/or low cell adsorption properties, the method including treating a surface of a metal medical device with a silane compound containing a functional group that is a polyoxyalkylene group, a metal salt-containing hydrophilic group, a halogen salt-containing hydrophilic group, or a fluorine-containing hydrophobic group.

The surface of a metal medical device may be treated with a silane compound by, for example, a method in which a solution prepared by mixing the silane compound with a solvent is applied to or sprayed on the metal medical device, or a method in which the metal medical device is immersed in the solution. The application, spraying, and immersion may be carried out by usually employed methods.

The solvent used in the preparation of the solution may be a solvent usually used in the above methods, and examples include water, perfluorohexane, acidic water, methanol, ethanol, and mixtures of water and methanol or ethanol. In particular, in the case of the silane compound containing a polyoxyalkylene group, a metal salt-containing hydrophilic group, or a halogen salt-containing hydrophilic group, suitable are water, acidic water, methanol, ethanol, and mixtures of water and methanol or ethanol. In the case of the silane compound containing a fluorine-containing hydrophobic group, suitable is perfluorohexane.

The concentration of the solution may be selected as appropriate depending on the method of treatment with the silane compound, the type of solution used in the treatment, and the like.

The solution preferably has a pH of 5 or lower. When the solution of the silane compound with a pH of 5 or lower is used to treat the surface of a metal medical device, the hydrolysis reaction can be accelerated. The pH is more preferably 4 or lower, while it is preferably 2 or higher, more preferably 3 or higher.

Thus, in another suitable embodiment of the present invention, the treatment step is carried out at a pH of 5 or lower.

The pH may be adjusted by any method, including conventional methods such as addition of an acid or alkali. Exemplary acids that can be used in the pH adjustment include inorganic acids such as sulfuric acid, nitric acid, and hydrochloric acid, and organic acids such as acetic acid. Exemplary alkalis include ammonia water, sodium hydroxide, and potassium hydroxide.

In the preparation method, the metal medical device may optionally be washed with acetone, ethanol, or the like and dried prior to the treatment step. The drying time and drying temperature may be selected as appropriate within the respective conventional ranges.

In the preparation method, a surface-treated metal medical device obtained after the treatment step may optionally be washed with water, acetone, ethanol, or the like and dried. The drying temperature and drying time may be selected as appropriate within the respective conventional ranges.

The preparation method includes holding a surface-treated metal medical device obtained through the treatment step at a humidity of 50% or higher. By holding the metal medical device surface-treated with the silane compound in an atmosphere at a humidity of 50% or higher, hydrolysis of the silane compound, a dehydration or deoxygenation condensation reaction between the silane compound and hydroxy groups on the surface of the metal medical device, and the like are further promoted so that the chemical bonding between hydroxy groups on the surface of the metal medical device and the silane compound becomes stronger. Therefore, the durability of the lubricant layer on the surface of the metal medical device can be further improved to further suppress deterioration of the sliding properties and low protein adsorption properties and/or low cell adsorption properties of the metal medical device having lubricity and low protein adsorption properties and/or low cell adsorption properties of the present invention. The humidity at which the surface-treated metal medical device is held is more preferably 60% or higher, still more preferably 80% or higher. The upper limit of the humidity is not particularly limited, and is preferably 100%, for example.

In the holding step, the time and temperature for holding the metal medical device at a humidity of 50% or higher may be appropriately selected so that the chemical bonding between hydroxy groups on the surface of the metal medical device and the silane compound becomes stronger to further suppress deterioration of the sliding properties and low protein adsorption properties and/or low cell adsorption properties of the metal medical device having lubricity and low protein adsorption properties and/or low cell adsorption properties of the present invention. The medical device is held for 20 to 60 hours, and is also preferably held at 20°C to 60°C.

### EXAMPLES

The present invention is specifically described by reference to examples below, but is not limited only to these examples.

### (Example 1)

A SUS guide wire (core wire) was washed with acetone and dried. The guide wire was immersed in a 10% by weight aqueous solution of SIM6492.57 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 2)

A SUS guide wire (core wire) was washed with acetone and dried. The guide wire was immersed in a 10% by weight aqueous solution of SIB1824.84 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 3)

A SUS guide wire (core wire) was washed with acetone and dried. The guide wire was immersed in a 10% by weight aqueous solution of SIT8402.0 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 4)

A SUS guide wire (core wire) was washed with acetone and dried. The guide wire was immersed in a 10% by weight aqueous solution of SIT8378.5 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 5)

A SUS guide wire (core wire) was washed with acetone and dried. The guide wire was immersed in a 10% by weight solution of 0.1% by weight OPTOOL DSX (available from Daikin Industries, Ltd.) in perfluorohexane for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 6)

A nickel-titanium alloy guide wire (core wire) was washed with acetone and dried. The guide wire was immersed in a 10% by weight aqueous solution of SIM6492.57 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 7)

A nickel-titanium alloy guide wire (core wire) was washed with acetone and dried. The guide wire was immersed in a 10% by weight aqueous solution of SIB1824.84 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 8)

A nickel-titanium alloy guide wire (core wire) was washed with acetone and dried. The guide wire was immersed in a 10% by weight aqueous solution of SIT8402.0 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 9)

A nickel-titanium alloy guide wire (core wire) was washed with acetone and dried. The guide wire was immersed in a 10% by weight aqueous solution of SIT8378.5 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 10)

A nickel-titanium alloy guide wire (core wire) was washed with acetone and dried. The guide wire was immersed in a 10% by weight solution of 0.1% by weight OPTOOL DSX (available from Daikin Industries, Ltd.) in perfluorohexane for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 11)

A SUS guide wire (core wire) was washed with acetone and dried. The guide wire was immersed in a 10% by weight aqueous solution of SIT8192.2 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 12)

A SUS guide wire (core wire) was washed with acetone and dried. The guide wire was immersed in a 10% by weight aqueous solution of SIB1500.0 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 13)

A SUS guide wire (core wire) was washed with acetone and dried. The guide wire was immersed for 30 minutes in a 10% by weight aqueous solution of SIB1500.0 (available from Gelest) adjusted to a pH of 4 with acetic acid, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Example 14)

A SUS guide wire (core wire) was washed with acetone and dried. The guide wire was immersed in a 10% by weight aqueous solution of SSP-060 (available from Gelest) for 30 minutes, and then taken out therefrom and allowed to stand for 24 hours at room temperature (25°C) and a humidity of 90%. The guide wire was subsequently washed with water and dried. The resulting surface-treated guide wire was evaluated for sliding properties as described later.

### (Comparative Example 1)

A SUS guide wire (core wire) only washed with acetone and dried was evaluated for sliding properties as described later.

### (Comparative Example 2)

A nickel-titanium alloy guide wire (core wire) only washed with acetone and dried was evaluated for sliding properties as described later.

### <Evaluation of sliding properties>

Water was put on the surface-treated guide wires and the guide wires, and each guide wire was then rubbed by a hand to evaluate sliding properties.

As a result of the evaluation, the surface-treated guide wires of Examples 1 to 5 and 11 to 14 were found to have a slippery surface and improved sliding properties as compared to the guide wire of Comparative Example 1. Particularly, the surface-treated guide wires of Example 11 and 14 had a highly slippery surface. Further, rubbing them 100 times gave no change in the slipperiness.

Similarly, the surface-treated guide wires of Examples 6 to 10 were found to have a slippery surface and improved sliding properties as compared to the guide wire of Comparative Example 2. Further, rubbing them 100 times gave no change in the slipperiness.

The following should be noted: It is known that polyoxyalkylene groups adsorb only a small amount of proteins or cells. Moreover, it is known that metal salt-containing hydrophilic groups adsorb a smaller amount of proteins or cells than the polyoxyalkylene groups. Furthermore, it is known that halogen salt-containing hydrophilic groups adsorb a slightly larger amount of proteins or cells than the metal salt-containing hydrophilic groups, but adsorb a smaller amount thereof than metals. In addition, it is known that fluorine-containing hydrophobic groups adsorb a relatively small amount of proteins or cells.

## Claims

1. A metal medical device, having lubricity and at least one of low protein adsorption properties or low cell adsorption properties,
the metal medical device having a surface at least partially treated with at least one silane compound selected from the group consisting of
a silane compound represented by the following formula (1),
a silane compound represented by the following formula (2),
a silane compound represented by the following formula (8),
a silane compound represented by the following formula (9),
a silane compound represented by the following formula (16),
a silane compound containing a linking unit A represented by the formula (17) and a linking unit B represented by the formula (18),
a silane compound represented by the following formula (23),
and
a silane compound represented by the following formula (24), 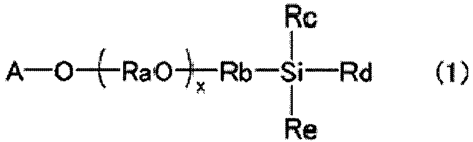 wherein A represents a hydrogen atom, a linear or branched C1-C3 alkyl group, or a linear or branched C1-C3 acyl group; Ra represents a linear or branched C1-C3 alkylene group; Rb represents a linear or branched C1-C5 alkylene group; Rc, Rd, and Re are the same as or different from one another, and each represent a halogen atom, a hydroxy group, a linear or branched C1-C3 alkyl group, or a linear or branched C1-C3 alkoxy group, provided that at least one of Rc, Rd, and Re is a halogen atom, a hydroxy group, or a linear or branched C1-C3 alkoxy group; and x represents the number of oxyalkylene repeating units each represented by RaO and is an integer of 2 to 500, 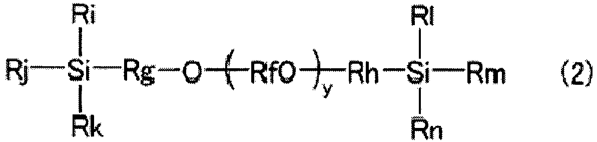 wherein Rf represents a linear or branched C1-C3 alkylene group; Rg and Rh are the same as or different from one another, and each represent a linear or branched C1-C5 alkylene group; Ri, Rj, Rk, Rl, Rm, and Rn are the same as or different from one another, and each represent a halogen atom, a hydroxy group, a linear or branched C1-C3 alkyl group, or a linear or branched C1-C3 alkoxy group, provided that at least one of Ri, Rj, Rk, Rl, Rm, and Rn is a halogen atom, a hydroxy group, or a linear or branched C1-C3 alkoxy group; and y represents the number of oxyalkylene repeating units each represented by RfO and is an integer of 2 to 500, 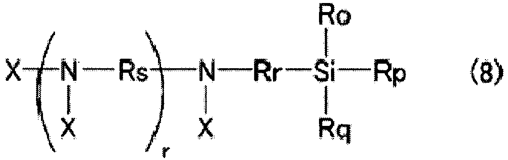 wherein Ro, Rp, and Rq are the same as or different from one another, and each represent a halogen atom, a hydroxy group, a linear or branched C1-C3 alkyl group, or a linear or branched C1-C3 alkoxy group, provided that at least one of Ro, Rp, and Rq is a halogen atom, a hydroxy group, or a linear or branched C1-C3 alkoxy group; Rr and Rs are the same as or different from one another, and each represent a linear or branched C1-C7 alkylene group; each X is the same or different and represents a metal salt-containing hydrophilic group; and r represents 0 or 1, 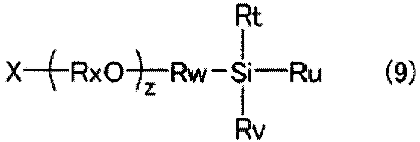 wherein Rt, Ru, and Rv are the same as or different from one another, and each represent a halogen atom, a hydroxy group, a linear or branched C1-C3 alkyl group, or a linear or branched C1-C3 alkoxy group, provided that at least one of Rt, Ru, and Rv is a halogen atom, a hydroxy group, or a linear or branched C1-C3 alkoxy group; Rw represents a linear or branched C1-C5 alkylene group; Rx represents a linear or branched C1-C3 alkylene group; each X is the same or different and represents a metal salt-containing hydrophilic group; and z represents the number of oxyalkylene repeating units each represented by RxO and is an integer of ***2*** to 500, 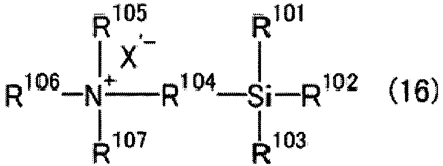 wherein R¹⁰¹, R¹⁰², and R¹⁰³ are the same as or different from one another, and each represent a halogen atom, a hydroxy group, a linear or branched C1-C3 alkyl group, or a linear or branched C1-C3 alkoxy group, provided that at least one of R¹⁰¹, R¹⁰², and R¹⁰³ is a halogen atom, a hydroxy group, or a linear or branched C1-C3 alkoxy group; R¹⁰⁴ represents a linear or branched C1-C7 alkylene group; R¹⁰⁵, R¹⁰⁶, and R¹⁰⁷ are the same as or different from one another, and each represent a hydrogen atom or a linear or branched C1-C5 alkyl group optionally containing an ether linkage; and X' represents a halogen atom, 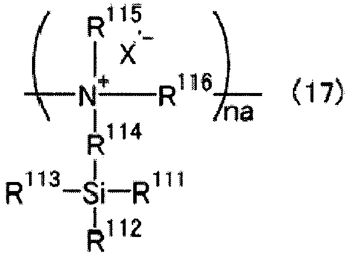 wherein R¹¹¹, R¹¹², and R¹¹³ are the same as or different from one another, and each represent a halogen atom, a hydroxy group, a linear or branched C1-C3 alkyl group, or a linear or branched C1-C3 alkoxy group, provided that at least one of R¹¹¹, R¹¹², and R¹¹³ is a halogen atom, a hydroxy group, or a linear or branched C1-C3 alkoxy group; R¹¹⁴ represents a linear or branched C1-C7 alkylene group; R¹¹⁵ represents a hydrogen atom or a linear or branched C1-C5 alkyl group optionally containing an ether linkage; R¹¹⁶ represents a linear or branched C1-C5 alkylene group; X' represents a halogen atom; and na represents an integer of 1 or more, 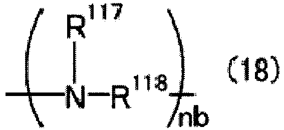 wherein R¹¹⁷ represents a hydrogen atom or a linear or branched C1-C5 alkyl group optionally containing an ether linkage; R¹¹⁸ represents a linear or branched C1-C5 alkylene group; and nb represents an integer of 1 or more, 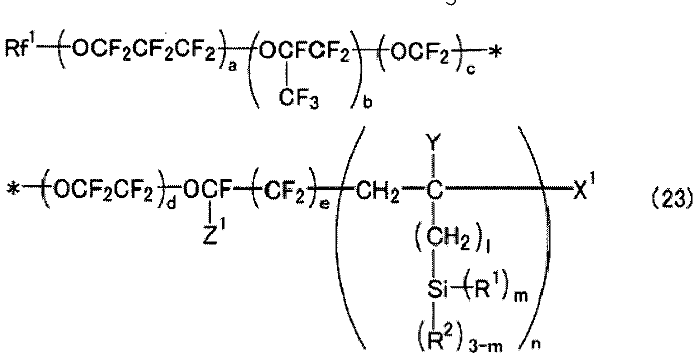 wherein Rf¹ represents a perfluoroalkyl group; Z¹ represents fluorine or a trifluoromethyl group; a, b, c, d, and e are the same as or different from one another, and each represent an integer of 0 or 1 or more, provided that a + b + c + d + e is 1 or more and the order of the repeating units parenthesized by subscripts a, b, c, d, and e occurring in the above formula is not limited to that shown; Y represents hydrogen or a C1-C4 alkyl group; X¹ represents hydrogen, bromine, or iodine; R¹ represents a hydroxy group or a hydrolyzable substituent; R² represents hydrogen or a monovalent hydrocarbon group; 1 represents 0, 1, or 2; m represents 1, 2, or 3; and n represents an integer of 1 or more; the two ends marked by * are directly bonded to each other, 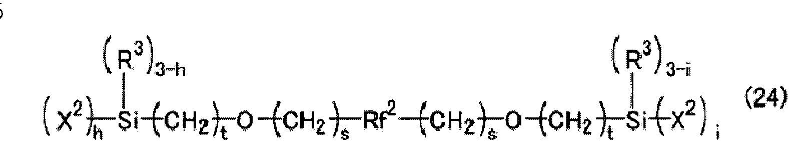 wherein Rf² represents a divalent group that contains a unit represented by -(CₖF₂ₖ)O- where k is an integer of 1 to 6, and has a non-branched linear perfluoropolyalkylene ether ) structure; each R³ is the same or different and represents a monovalent C1-C8 hydrocarbon group; each X² is the same or different and represents a hydrolyzable group or a halogen atom; each s is the same or different and represents an integer of 0 to 2; each t is the same or different and represents an integer ) of 1 to 5; and h and i are the same as or different from one another, and each represent 2 or 3.

2. A method for preparing a metal medical device having lubricity and at least one of low protein adsorption properties or low cell adsorption properties,
the method comprising treating a surface of a metal medical device with a silane compound containing a functional group that is a polyoxyalkylene group, a metal salt-containing hydrophilic group, a halogen salt-containing hydrophilic group, or a fluorine-containing hydrophobic group, further comprising holding a surf ace-treated metal medical device obtained through the treatment step at a humidity of 50% or higher for 20 to 60 hours.

3. The method according to claim 2,
wherein the treatment step is carried out at a pH of 5 or lower.

## Patentansprüche

1. Medizinische Metallvorrichtung, welche Gleitfähigkeit und mindestens eines von niedrigen Proteinadsorptionseigenschaften oder niedrigen Zelladsorptionseigenschaften aufweist,
wobei die medizinische Metallvorrichtung eine Oberfläche aufweist, die zumindest teilweise mit zumindest einer Silanverbindung behandelt ist, die ausgewählt ist aus der Gruppe bestehend aus
einer durch die folgende Formel (1) dargestellten Silanverbindung,
einer durch die folgende Formel (2) dargestellten Silanverbindung,
einer durch die folgende Formel (8) dargestellten Silanverbindung,
einer durch die folgende Formel (9) dargestellten Silanverbindung,
einer durch die folgende Formel (16) dargestellten Silanverbindung,
einer Silanverbindung, die eine durch die Formel (17) dargestellte Verknüpfungseinheit A und eine durch die Formel (18) dargestellte Verknüpfungseinheit B enthält,
einer durch die folgende Formel (23) dargestellten Silanverbindung und einer durch die folgende Formel (24) dargestellten Silanverbindung, 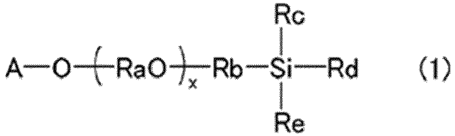 wobei A ein Wasserstoffatom, eine lineare oder verzweigte C1-C3-Alkylgruppe oder eine lineare oder verzweigte C1-C3-Acylgruppe darstellt; Ra eine lineare oder verzweigte C1-C3-Alkylengruppe darstellt; Rb eine lineare oder verzweigte C1-C5-Alkylengruppe darstellt; Rc, Rd und Re gleich oder voneinander verschieden sind und jeweils ein Halogenatom, eine Hydroxygruppe, eine lineare oder verzweigte C1-C3-Alkylgruppe oder eine lineare oder verzweigte C1-C3-Alkoxygruppe darstellen, unter der Voraussetzung, dass mindestens eines von Rc, Rd und Re ein Halogenatom, eine Hydroxygruppe oder eine lineare oder verzweigte C1-C3-Alkoxygruppe ist; und x die Anzahl an jeweils durch RaO dargestellten Oxyalkylen-Wiederholungseinheiten darstellt und eine ganze Zahl von 2 bis 500 ist, 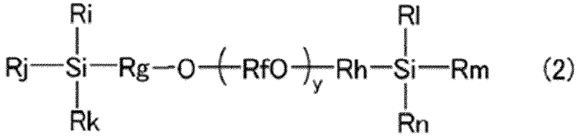 wobei Rf eine lineare oder verzweigte C1-C3-Alkylengruppe darstellt; Rg und Rh gleich oder voneinander verschieden sind und jeweils eine lineare oder verzweigte C1-C5-Alkylengruppe darstellen; Ri, Rj, Rk, Rl, Rm und Rn gleich oder voneinander verschieden sind und jeweils ein Halogenatom, eine Hydroxygruppe, eine lineare oder verzweigte C1-C3-Alkylgruppe oder eine lineare oder verzweigte C1-C3-Alkoxygruppe darstellen, unter der Voraussetzung, dass mindestens eines von Ri, Rj, Rk, Rl, Rm und Rn ein Halogenatom, eine Hydroxygruppe oder eine lineare oder verzweigte C1-C3-Alkoxygruppe ist; und y die Anzahl an jeweils durch RfO dargestellten Oxyalkylen-Wiederholungseinheiten darstellt und eine ganze Zahl von 2 bis 500 ist, 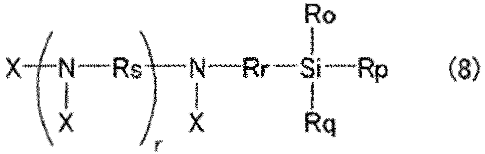 wobei Ro, Rp und Rq gleich oder voneinander verschieden sind und jeweils ein Halogenatom, eine Hydroxygruppe, eine lineare oder verzweigte C1-C3-Alkylgruppe oder eine lineare oder verzweigte C1-C3-Alkoxygruppe darstellen, unter der Voraussetzung, dass mindestens eines von Ro, Rp und Rq ein Halogenatom, eine Hydroxygruppe oder eine lineare oder verzweigte C1-C3-Alkoxygruppe ist; Rr und Rs gleich oder voneinander verschieden sind und jeweils eine lineare oder verzweigte C1-C7-Alkylengruppe darstellen; jedes X gleich oder verschieden ist und eine metallsalzhaltige hydrophile Gruppe darstellt; und r für 0 oder 1 steht, 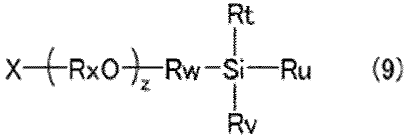 wobei Rt, Ru und Rv gleich oder voneinander verschieden sind und jeweils ein Halogenatom, eine Hydroxygruppe, eine lineare oder verzweigte C1-C3-Alkylgruppe oder eine lineare oder verzweigte C1-C3-Alkoxygruppe darstellen, unter der Voraussetzung, dass mindestens eines von Rt, Ru und Rv ein Halogenatom, eine Hydroxygruppe oder eine lineare oder verzweigte C1-C3-Alkoxygruppe ist; Rw eine lineare oder verzweigte C1-C5-Alkylengruppe darstellt; Rx eine lineare oder verzweigte C1-C3-Alkylengruppe darstellt; jedes X gleich oder verschieden ist und eine metallsalzhaltige hydrophile Gruppe darstellt; und z die Anzahl an jeweils durch RxO dargestellten Oxyalkylen-Wiederholungseinheiten darstellt und eine ganze Zahl von 2 bis 500 ist, 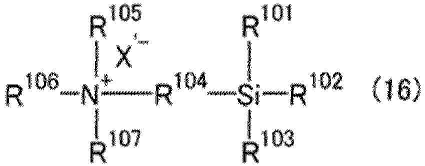 wobei R¹⁰¹, R¹⁰² und R¹⁰³ gleich oder voneinander verschieden sind und jeweils ein Halogenatom, eine Hydroxygruppe, eine lineare oder verzweigte C1-C3-Alkylgruppe oder eine lineare oder verzweigte C1-C3-Alkoxygruppe darstellen, unter der Voraussetzung, dass mindestens eines von R¹⁰¹, R¹⁰² und R¹⁰³ ein Halogenatom, eine Hydroxygruppe oder eine lineare oder verzweigte C1-C3-Alkoxygruppe ist; R¹⁰⁴ eine lineare oder verzweigte C1-C7-Alkylengruppe darstellt; R¹⁰⁵, R¹⁰⁶ und R¹⁰⁷ gleich oder voneinander verschieden sind und jeweils ein Wasserstoffatom oder eine lineare oder verzweigte C1-C5-Alkylgruppe darstellen, die optional eine Etherverknüpfung enthält; und X' ein Halogenatom darstellt, 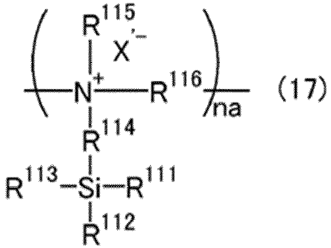 wobei R¹¹¹, R¹¹², und R¹¹³ gleich oder voneinander verschieden sind und jeweils ein Halogenatom, eine Hydroxygruppe, eine lineare oder verzweigte C1-C3-Alkylgruppe oder eine lineare oder verzweigte C1-C3-Alkoxygruppe darstellen, unter der Voraussetzung, dass mindestens eines von R¹¹¹, R¹¹² und R¹¹³ ein Halogenatom, eine Hydroxygruppe oder eine lineare oder verzweigte C1-C3-Alkoxygruppe ist; R¹¹⁴ eine lineare oder verzweigte C1-C7-Alkylengruppe darstellt; R¹¹⁵ ein Wasserstoffatom oder eine lineare oder verzweigte C1-C5-Alkylgruppe darstellt, die optional eine Etherverknüpfung enthält; R¹¹⁶ eine lineare oder verzweigte C1-C5-Alkylengruppe darstellt; X' ein Halogenatom darstellt; und na eine ganze Zahl von 1 oder mehr darstellt, 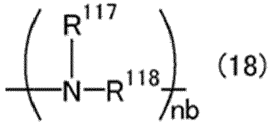 wobei R¹¹⁷ ein Wasserstoffatom oder eine lineare oder verzweigte C1-C5-Alkylgruppe darstellt, die optional eine Etherverknüpfung enthält; R¹¹⁸ eine lineare oder verzweigte C1-C5-Alkylengruppe darstellt; und nb eine ganze Zahl von 1 oder mehr darstellt, 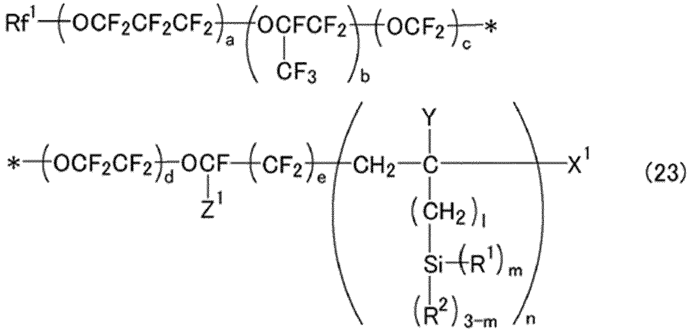 wobei Rf¹ eine Perfluoralkylgruppe darstellt; Z¹ Fluor oder eine Trifluormethylgruppe darstellt; a, b, c, d und e gleich oder voneinander verschieden sind und jeweils eine ganze Zahl von 0 oder 1 oder mehr darstellen, unter der Voraussetzung, dass a + b + c + d + e 1 oder mehr ist und die Reihenfolge der mit den tiefgestellten Zeichen a, b, c, d und e in Klammern gesetzten Wiederholungseinheiten, die in der obigen Formel vorkommen, nicht auf diejenige beschränkt ist, die gezeigt ist; Y Wasserstoff oder eine C1-C4-Alkylgruppe darstellt; X¹ Wasserstoff, Brom oder Iod darstellt; R¹ eine Hydroxygruppe oder einen hydrolysierbaren Substituenten darstellt; R² Wasserstoff oder eine einwertige Kohlenwasserstoffgruppe darstellt; I 0, 1 oder 2 darstellt; m 1, 2 oder 3 darstellt; und n eine ganze Zahl von 1 oder mehr darstellt; die beiden mit * markierten Enden direkt miteinander verbunden sind, 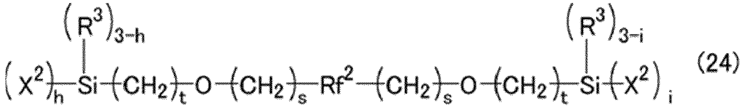 wobei Rf² eine zweiwertige Gruppe darstellt, die eine durch - (CₖF₂ₖ) O - dargestellte Einheit enthält, worin k eine ganze Zahl von 1 bis 6 ist, und die eine unverzweigte lineare Perfluorpolyalkylenetherstruktur aufweist; jedes R³ gleich oder verschieden ist und eine einwertige C1-C8-Kohlenwasserstoffgruppe darstellt; jedes X² gleich oder verschieden ist und eine hydrolysierbare Gruppe oder ein Halogenatom darstellt; jedes s gleich oder verschieden ist und eine ganze Zahl von 0 bis 2 darstellt; jedes t gleich oder verschieden ist und eine ganze Zahl von 1 bis 5 darstellt; und h und i gleich oder voneinander verschieden sind und jeweils 2 oder 3 darstellen.

2. Verfahren zur Herstellung einer medizinischen Metallvorrichtung, welche Gleitfähigkeit und zumindest eines von niedrigen Proteinadsorptionseigenschaften oder niedrigen Zelladsorptionseigenschaften aufweist,
wobei das Verfahren das Behandeln einer Oberfläche einer medizinischen Metallvorrichtung mit einer Silanverbindung umfasst, welche eine funktionelle Gruppe enthält, die eine Polyoxyalkylengruppe, eine metallsalzhaltige hydrophile Gruppe, eine halogensalzhaltige hydrophile Gruppe oder eine fluorhaltige hydrophobe Gruppe ist, welches zudem umfasst, dass eine durch den Behandlungsschritt erhaltene oberflächenbehandelte medizinische Metallvorrichtung für 20 bis 60 Stunden bei einer Luftfeuchtigkeit von 50% oder höher gehalten wird.

3. Verfahren nach Anspruch 2,
wobei der Behandlungsschritt bei einem pH von 5 oder niedriger durchgeführt wird.

## Revendications

1. Dispositif médical métallique, ayant une lubricité et au moins une des caractéristiques de faible adsorption de protéines ou de faible adsorption de cellules,
le dispositif médical métallique ayant une surface au moins partiellement traitée avec au moins un composé silane choisi dans le groupe constitué
d'un composé silane représenté par la formule suivante (1),
d'un composé silane représenté par la formule suivante (2),
d'un composé silane représenté par la formule suivante (8),
d'un composé silane représenté par la formule suivante (9),
d'un composé silane représenté par la formule suivante (16),
d'un composé silane contenant une unité de liaison A représentée par la formule (17) et une unité de liaison B représentée par la formule (18),
d'un composé silane représenté par la formule suivante (23), et
d'un composé silane représenté par la formule suivante (24) ; 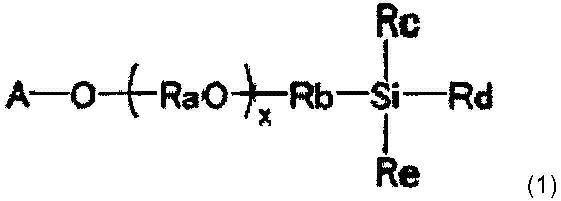 où A représente un atome d'hydrogène, un groupement alkyle en C1 à C3 linéaire ou ramifié, ou un groupement acyle en C1 à C3 linéaire ou ramifié ; Ra représente un groupement alcylène en C1 à C3 linéaire ou ramifié ; Rb représente un groupement alcylène en C1 à C5 linéaire ou ramifié ; Rc, Rd et Re sont identiques ou différents les uns des autres, et chacun représente un atome d'halogène, un groupement hydroxy, un groupement alkyle en C1 à C3 linéaire ou ramifié, ou un groupement alcoxy en C1 à C3 linéaire ou ramifié, pourvu que l'au moins un parmi Rc, Rd et Re soit un atome d'halogène, un groupement hydroxy, ou un groupement alcoxy en C1 à C3 linéaire ou ramifié ; et x représente le nombre d'unités de répétition oxyalcylène, chacune représentée par RaO, et est un nombre entier de 2 à 500, 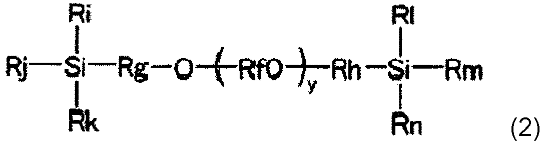 où Rf représente un groupement alcylène en C1 à C3 linéaire ou ramifié ; Rg et Rh sont identiques ou différents les uns des autres, et chacun représente un groupement alcylène en C1 à C5 linéaire ou ramifié ; Ri, Rj, Rk, Rl, Rm et Rn sont identiques ou différents les uns des autres, et chacun représente un atome d'halogène, un groupement hydroxy, un groupement alkyle en C1 à C3 linéaire ou ramifié, ou un groupement alcoxy en C1 à C3 linéaire ou ramifié, pourvu que l'un au moins parmi Ri, Rj, Rk, Rl, Rm et Rn soit un atome d'halogène, un groupement hydroxy, ou un groupement alcoxy en C1 à C3 linéaire ou ramifié ; et y représente le nombre d'unités de répétition oxyalcylène, chacune représentée par RfO, et est un nombre entier de 2 à 500, 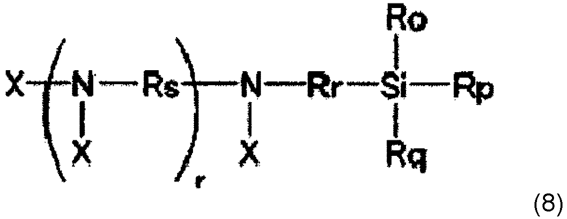 où Ro, Rp et Rq sont identiques ou différents les uns des autres, et chacun représente un atome d'halogène, un groupement hydroxy, un groupement alkyle en C1 à C3 linéaire ou ramifié, ou un groupement alcoxy en C1 à C3 linéaire ou ramifié, pourvu que l'un au moins parmi Ro, Rp et Rq soit un atome d'halogène, un groupement hydroxy, ou un groupement alcoxy en C1 à C3 linéaire ou ramifié ; Rr et Rs sont identiques ou différents les uns des autres, et chacun représente un groupement alcylène en C1 à C7 linéaire ou ramifié ; tous les X sont identiques ou différents et représentent un groupement hydrophile contenant un sel métallique ; et r représente 0 ou 1, 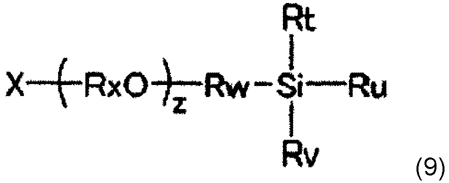 où Rt Ru et Rv sont identiques ou différents les uns des autres, et chacun représente un atome d'halogène, un groupement hydroxy, un groupement alkyle en C1 à C3 linéaire ou ramifié, ou un groupement alcoxy en C1 à C3 linéaire ou ramifié, pourvu que l'un au moins parmi Rt, Ru et Rv soit un atome d'halogène, un groupement hydroxy, ou un groupement alcoxy en C1 à C3 linéaire ou ramifié ; Rw représente un groupement alcylène en C1 à C5 linéaire ou ramifié ; Rx représente un groupement alcylène en C1 à C3 linéaire ou ramifié ; tous les X sont identiques ou différents et représentent un groupement hydrophile contenant un sel métallique ; et z représente le nombre d'unités de répétition oxyalcylène, chacune représentée par RxO, et est un nombre entier de 2 à 500, 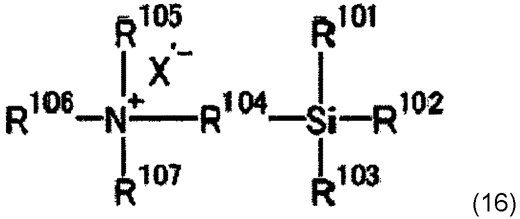 où R¹⁰¹, R¹⁰² et R¹⁰³ sont identiques ou différents les uns des autres, et chacun représente un atome d'halogène, un groupement hydroxy, un groupement alkyle en C1 à C3 linéaire ou ramifié, ou un groupement alcoxy en C1 à C3 linéaire ou ramifié, pourvu que l'un au moins parmi R¹⁰¹, R¹⁰² et R¹⁰³ soit un atome d'halogène, un groupement hydroxy, ou un groupement alcoxy en C1 à C3 linéaire ou ramifié ; R¹⁰⁴ représente un groupement alcylène en C1 à C7 linéaire ou ramifié ; R¹⁰⁵, R¹⁰⁶ et R¹⁰⁷ sont identiques ou différents les uns des autres, et chacun représente un atome d'hydrogène ou un groupement alkyle en C1 à C5 linéaire ou ramifié, contenant éventuellement une liaison éther; et X' représente un atome d'halogène, 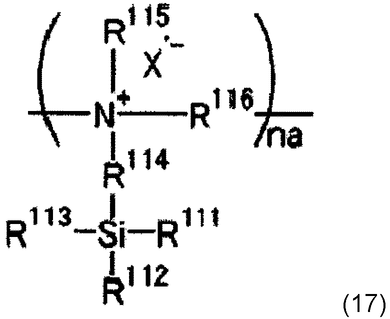 où R^{1U}, R¹¹² et R¹¹³ sont identiques ou différents les uns des autres, et chacun représente un atome d'halogène, un groupement hydroxy, un groupement alkyle en C1 à C3 linéaire ou ramifié, ou un groupement alcoxy en C1 à C3 linéaire ou ramifié, pourvu que l'un au moins parmi R¹¹¹, R¹¹² et R¹¹³ soit un atome d'halogène, un ^{groupement} hydroxy, ou un groupement alcoxy en C1 à C3 linéaire ou ramifié ; R¹¹⁴ représente un groupement alcylène en C1 à C7 linéaire ou ramifié ; R¹¹⁵ représente un atome d'hydrogène ou un groupement alkyle en C1 à C5 linéaire ou ramifié contenant éventuellement une liaison éther ; R¹¹⁶ représente un groupement alcylène en C1 à C5 linéaire ou ramifié ; X' représente un atome d'halogène; et na représente un nombre entier égal à 1 ou plus, 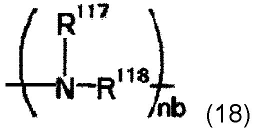 où R¹¹⁷ représente un atome d'hydrogène ou un groupement alkyle en C1 à C5 linéaire ou ramifié contenant éventuellement une liaison éther ; R¹¹⁸ représente un groupement alcylène en C1 à C5 linéaire ou ramifié ; et nb représente un nombre entier égal à 1 ou plus, 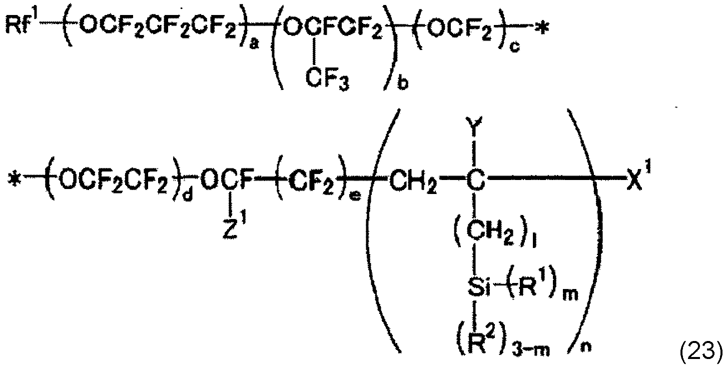 où Rf¹ représente un groupement perfluoro alkyle ; Z¹ représente du fluor ou un groupement trifluoro méthyle ; a, b, c, d, et e sont identiques ou différents les uns des autres, et chacun représente un nombre entier égal à 0 ou 1 ou plus, pourvu que a + b + c + d + e soit égal à 1 ou plus, et l'ordre des unités de répétition entre parenthèses avec les indices a, b, c, d et e reproduit dans la formule ci-dessus n'est pas limité à celui qui est présenté ; Y représente un atome d'hydrogène ou un groupement alkyle en C1 à C4 ; X¹ représente un atome d'hydrogène, de brome ou d'iode ; R¹ représente un groupement hydroxy ou un substituant hydrolysable ; R² représente un atome d'hydrogène ou un groupement hydrocarboné monovalent ; I représente 0, 1, ou 2 ; m représente 1, 2 ou 3 ; et n représente un nombre entier égal à 1 ou plus ; les deux extrémités repérées par * sont directement liées l'une à l'autre, 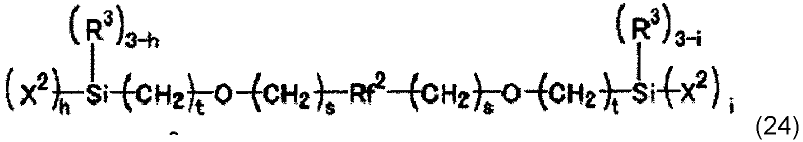 où Rf² représente un groupement divalent qui contient une unité représentée par -(CₖF₂ₖ)O-, où k est un nombre entier de 1 à 6, et a une structure d'éther de perfluoro poly alcylène linéaire non ramifiée ; tous les R³ sont identiques ou différents et représentent un groupement hydrocarboné monovalent en C1 à C8 ; tous les X² sont identiques ou différents et représentent un groupement hydrolysable ou un atome d'halogène ; tous les s sont identiques ou différents et représentent un nombre entier de 0 à 2 ; tous les t sont identiques ou différents et représentent un nombre entier de 1 à 5 ; et h et i sont identiques ou différents l'un de l'autre, et chacun représente 2 ou 3.

2. Procédé de préparation d'un dispositif médical métallique ayant lubricité et au moins une des caractéristiques de faible adsorption de protéines ou de faible adsorption de cellules,
le procédé comprenant le traitement d'une surface d'un dispositif médical métallique avec un composé silane contenant un groupement fonctionnel qui est un groupement poly oxyalcylène, un groupement hydrophile contenant un sel métallique, un groupement hydrophile contenant un sel halogéné, ou un groupement hydrophobe contenant du fluor, comprenant en outre le maintien d'un dispositif médical métallique à la surface traitée obtenu par l'étape de traitement à une humidité de 50 % ou plus pendant 20 à 60 heures.

3. Procédé selon la revendication 2, dans lequel l'étape de traitement est réalisée à un pH de 5 ou moins.
